(19) Europäisches Patentamt
European Patent Office
Office européen des brevets

(11) EP 4 302 757 A1

(12) EUROPEAN PATENT APPLICATION

(43) Date of publication:
**10.01.2024 Bulletin 2024/02**

(21) Application number: **22183652.1**

(22) Date of filing: **07.07.2022**

(51) International Patent Classification (IPC):
***A61K 31/122*** (2006.01) ***A61K 31/136*** (2006.01)
***A61K 31/137*** (2006.01) ***A61P 17/00*** (2006.01)

(52) Cooperative Patent Classification (CPC):
**A61K 31/122; A61K 31/136; A61K 31/137; A61P 17/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Christian-Albrechts-Universität zu Kiel**
  **24118 Kiel (DE)**
- **Helmholtz-Zentrum für Ozeanforschung Kiel (GEOMAR)**
  **24148 Kiel (DE)**

(72) Inventors:
- **Peifer, Christian**
  **24118 Kiel (DE)**
- **Jansen, Björn**
  **24118 Kiel (DE)**
- **Tasdemir, Deniz**
  **24106 Keil (DE)**
- **Wenzel-Storjohann, Arlette**
  **24106 Kiel (DE)**

(74) Representative: **Taruttis, Stefan Georg**
**TARUTTIS Patentanwaltskanzlei**
**Aegidientorplatz 2b**
**30159 Hannover (DE)**

(54) **PHARMACEUTICAL COMPOUNDS FOR TREATMENT OF ONYCHOMYCOSIS**

(57) The present invention relates to pharmaceutical compounds for use in the treatment of onychomycoses, which compounds have the advantage of good penetration into keratin, e.g. into fingernails and toenails of humans, and into hooves of animals. The compound for medical treatment of onychomycoses, including use in the treatment of onychomycoses in photodynamic treatment comprises
structure I:

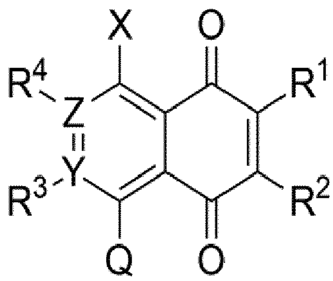

EP 4 302 757 A1

## Description

The present invention relates to pharmaceutical compounds for use in the treatment of onychomycoses, which compounds have the advantage of good penetration into keratin, e.g. into fingernails and toenails of humans, and into hooves of animals. Further, the pharmaceutical compounds have the advantage of effectively inactivating both vegetative cells and spores of mycosis that is present within the keratin of fingernails, toenails or hooves.

A further advantage of the pharmaceutical compounds of the invention is that they show little or no penetration into the nail matrix underlying the nail or hoof, so that the anti-mycotic activity of the compounds is generally limited to the nail or hoof.

The pharmaceutical compounds are especially suitable for use in photodynamic treatment of onychomycosis, the therapy comprising the steps of applying one of the compounds onto a nail or hoof, the compound migrating into the nail or hoof, and irradiating the nail or hoof. Under irradiation, the pharmaceutical compounds with in the nail or hoof generate reactive oxygen species (ROS), which effectively inactivate both vegetative cells and spores within the nail or hoof. Accordingly, the pharmaceutical compounds have the advantage of fast inactivation of the onychomycosis.

Onychomycoses are e.g. caused by Trichophyton rubrum and/or Candida albicans.

## State of the art

Pharmaceutical compounds for use in mycosis, e.g. Clotrimazol or Terbinafin, selectively inhibit the pathway of ergosterol synthesis, which is specific for mycosis but does not occur in humans.

A disadvantage of pharmaceutical compounds used in antimycotic therapy is that only vegetative cells of the mycosis are attacked, allowing spores to persist. Further, such compounds often have low penetration into a nail, resulting in low efficacy of the treatment of onychomycosis, and the necessity for daily application for several months, with high relapse rates.

EP 200219 B1 describes processes for preparing derivatives of naphthazarin.

Papageorgiou et al., Angew. Chem Int. Ed. 270-300 (1999) gives an overview of naphthazarin compounds and their pharmaceutical uses.

## Object of the invention

It is an object of the invention to provide pharmaceutical compounds for use in the treatment of onychomycoses that allow for inactivation of both vegetative cells and spores of the mycosis. A further object is to provide pharmaceutical compounds that have high penetration rates into nail and hooves. A preferred object is to provide pharmaceutical compounds that allow for fast inactivation of onychomycosis, more preferably without impairing the nail matrix of the patient.

## Description of the invention

The invention achieves the object by the features of the claims, especially by providing a pharmaceutical compound for use in medical treatment, e.g. in the medical treatment of fungal infections, preferably for use in the treatment of onychomycoses, including use in the treatment of onychomycoses by spore-forming microorganisms, e.g. by spore-forming fungi, especially in photodynamic treatment, preferably comprising the step of irradiation at a wavelength of 450 to 700 nm, e.g. 500 to 600 nm, after topical application of the compound to the nail, the pharmaceutical compound comprising or consisting of structure I:

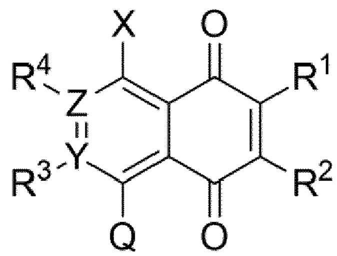

(Structure I),

wherein Z is one of C and N,

Y is one of C and N,
X is OH or SH or NHR, or less preferred $NR_2$,
Q is OH or SH or NHR, or less preferred $NR_2$,
preferably X is OH and Q is OH, and each R, R1, R2, R3 and R4 is independently a hydrogen, a halogen, e.g. Cl, I, or Br, preferably I, a $C_1$ to $C_{12}$ linear, branched or cyclic alkyl or aryl, e.g. methyl, ethyl, n-propyl, isopropyl, n-butyl, isobutyl, n-pentyl, 2-pentyl, n-hexyl, 2-hexyl, 3-hexyl, cyclohexyl, heptyl, octyl, nonyl, decyl, undecyl or dodecyl, propylenyl, butylenyl, pentylenyl, hexenyl, or a phenyl or benzyl.

Preferred groups for R, R1, R2, R3 and R4 are those of

Table 1:

| number | structure | number | structure | number | structure |
|---|---|---|---|---|---|
| 1 | 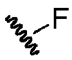 | 2 | 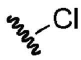 | 3 | 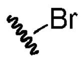 |
| 4 | 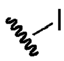 | 5 | 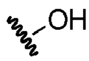 | 6 | 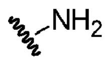 |
| 7 | 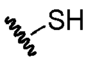 | 8 | 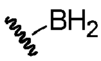 | | |
| chain length = $C_1$ | | | | | |
| 9 | 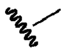 | 10 | 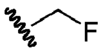 | 11 | 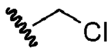 |
| 12 | 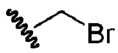 | 13 | 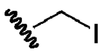 | 14 | 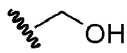 |
| 15 | 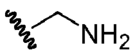 | 16 | 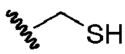 | 17 | 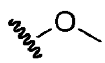 |
| 18 | 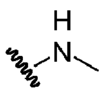 | 19 | 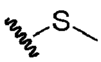 | 20 | 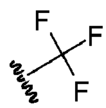 |
| 21 | 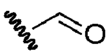 | | | | |
| chain length = $C_2$ | | | | | |
| 22 | 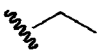 | 23 | 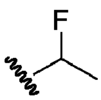 | 24 | 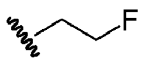 |
| 25 | 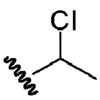 | 26 | 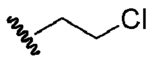 | 27 | 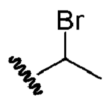 |
| 28 | 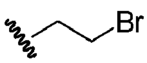 | 29 | 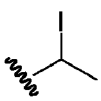 | 30 | 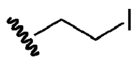 |
| 31 | 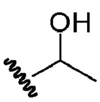 | 32 | 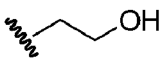 | 33 | 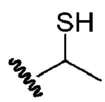 |

(continued)

| | | | | | |
|---|---|---|---|---|---|
| chain length = $C_2$ | | | | | |
| 34 | 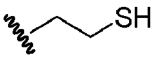 | 35 | 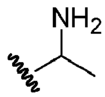 | 36 | 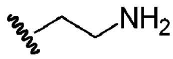 |
| 37 | 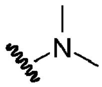 | 38 | 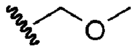 | 39 | 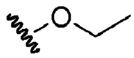 |
| 40 | 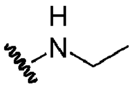 | 41 | 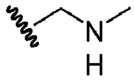 | 42 | 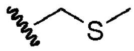 |
| 43 | 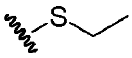 | 44 | 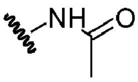 | 45 | 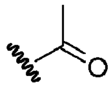 |
| 46 | 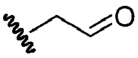 | 47 | 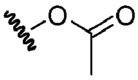 | | |
| chain length = $C_3$ | | | | | |
| 48 | 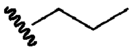 | 49 | 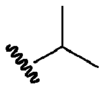 | 50 | 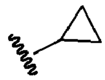 |
| 51 | 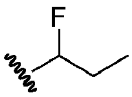 | 52 | 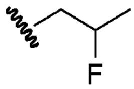 | 53 | 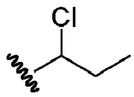 |
| 54 | 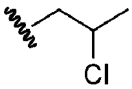 | 55 | 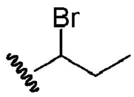 | 56 | 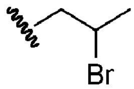 |
| 57 | 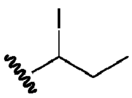 | 58 | 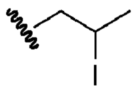 | 59 | 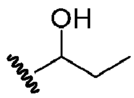 |
| 60 | 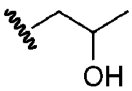 | 61 | 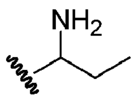 | 62 | 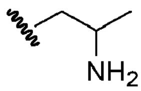 |
| 63 | 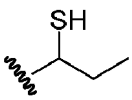 | 64 | 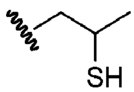 | 65 | 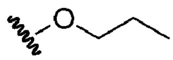 |
| 66 | 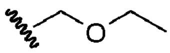 | 67 | 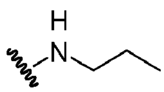 | 68 | 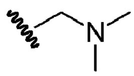 |
| 69 | 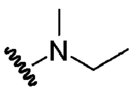 | 70 | 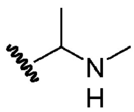 | 71 | 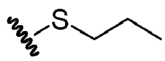 |
| 72 | 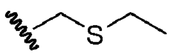 | 73 | 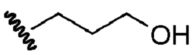 | 74 | |

(continued)

| | | | | | |
|---|---|---|---|---|---|
| chain length = $\mathbf{C_3}$ | | | | | |
| 75 | 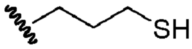 | 76 | 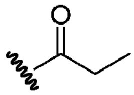 | 77 | 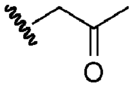 |
| 78 | 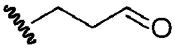 | 79 | 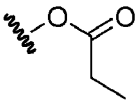 | 80 | 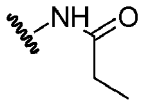 |
| 81 | 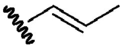 | 82 | 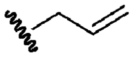 | | |
| chain length = $\mathbf{C_4}$ | | | | | |
| 83 | 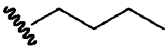 | 84 | 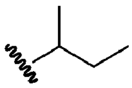 | 85 | 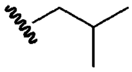 |
| 86 | 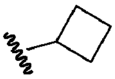 | 87 | 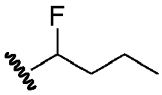 | 88 | 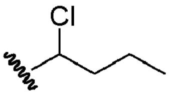 |
| 89 | 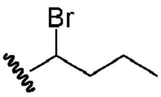 | 90 | 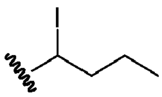 | 91 | 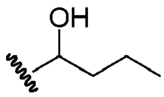 |
| 92 | 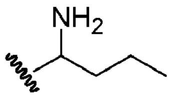 | 93 | 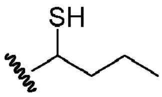 | 94 | 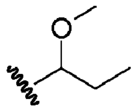 |
| 95 | 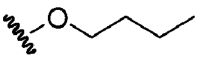 | 96 | 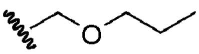 | 97 | 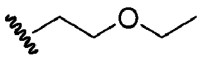 |
| 98 | 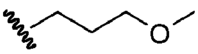 | 99 | 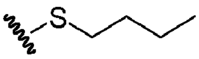 | 100 | 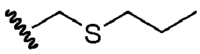 |
| 101 | 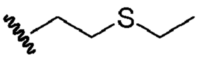 | 102 | 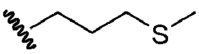 | 103 | 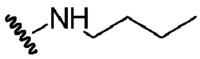 |
| 104 | 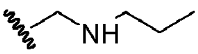 | 105 | 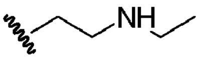 | 106 | 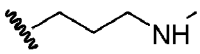 |
| 107 | 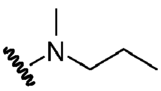 | 108 | 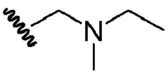 | 109 | 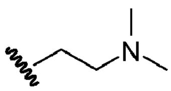 |
| 110 | 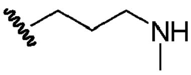 | 111 | 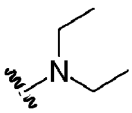 | 112 | 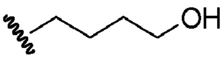 |
| 113 | 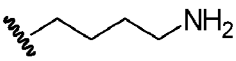 | 114 | 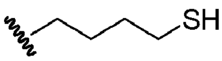 | 115 | |
| 116 | | 117 | | 118 | |

(continued)

| | | | | | |
|---|---|---|---|---|---|
| chain length = $C_4$ | | | | | |
| 119 | 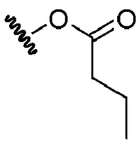 | 120 | 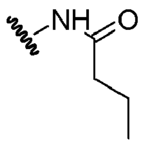 | 121 | 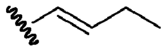 |
| 122 | 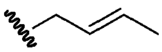 | 123 | 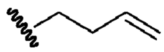 | 124 | 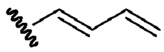 |
| chain length = $C_5$ | | | | | |
| 125 | 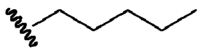 | 126 | 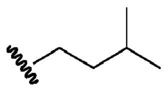 | 127 | 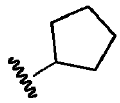 |
| 128 | 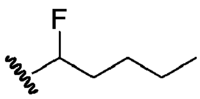 | 129 | 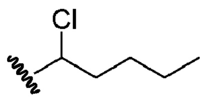 | 130 | 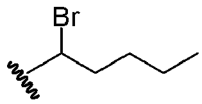 |
| 131 | 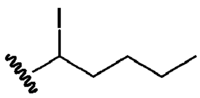 | 132 | 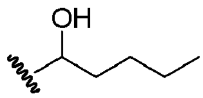 | 133 | 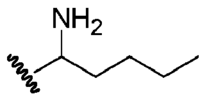 |
| 134 | 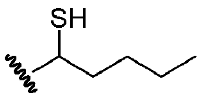 | 135 | 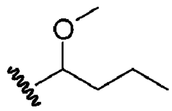 | 136 | 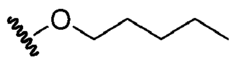 |
| 137 | 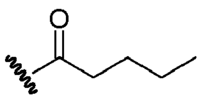 | 138 | 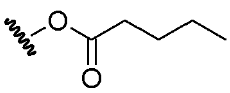 | 139 | 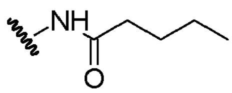 |
| 140 | 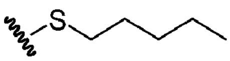 | 141 | 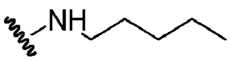 | 142 | 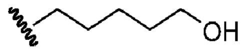 |
| 143 | 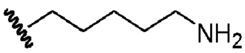 | 144 | 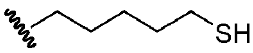 | 145 | 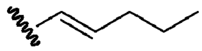 |
| 146 | 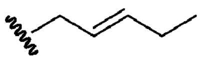 | 147 | 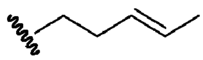 | 148 | 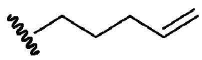 |
| 149 | 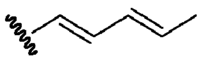 | 150 | 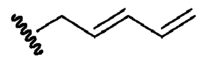 | 151 | 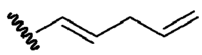 |
| 152 | 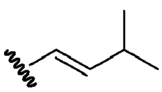 | 153 | 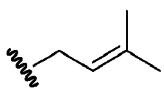 | 154 | 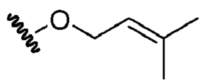 |
| chain length = $C_6$ | | | | | |
| 155 | 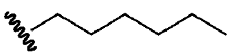 | 156 | 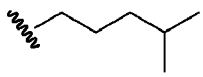 | 157 | 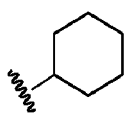 |
| 158 | 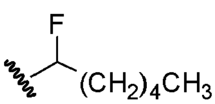 | 159 | | 160 | |

(continued)

| | | | | | |
|---|---|---|---|---|---|
| chain length = **$C_6$** | | | | | |
| 161 | 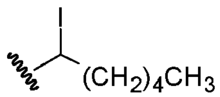 | 162 | 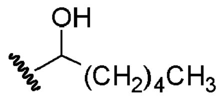 | 163 | 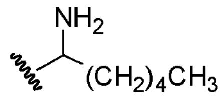 |
| 164 | 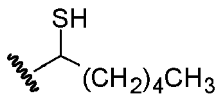 | 165 | 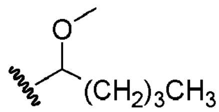 | 166 | 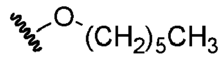 |
| 167 | 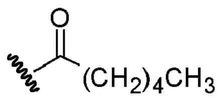 | 168 | 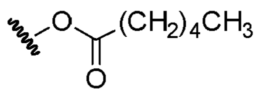 | 169 | 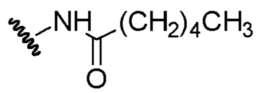 |
| 170 | 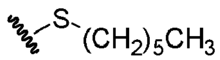 | 171 | 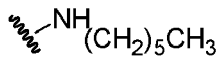 | 172 | $CH_2-(CH_2)_5OH$ |
| 173 | $CH_2-(CH_2)_5NH_2$ | 174 | $CH_2-(CH_2)_5SH$ | 175 | 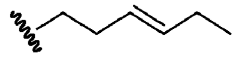 |
| 176 | 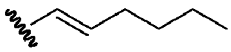 | 177 | 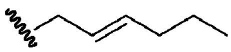 | 178 | 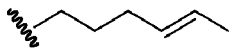 |
| 179 | 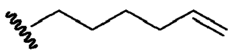 | 180 | 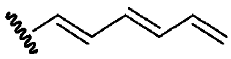 | 181 | 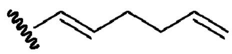 |
| 182 | 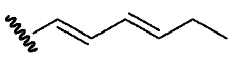 | 183 | 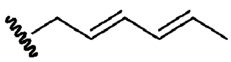 | 184 | 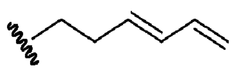 |
| 185 | 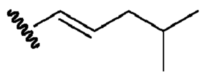 | 186 | 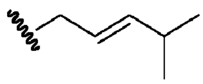 | 187 | 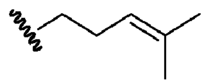 |
| 188 | 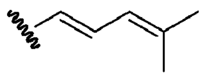 | | | | |
| chain length = **$C_7$** | | | | | |
| 189 | 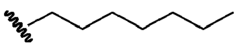 | 190 | 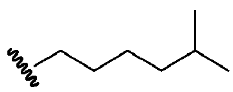 | 191 | 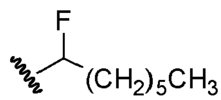 |
| 192 | 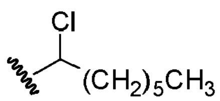 | 193 | 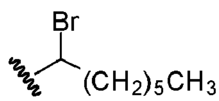 | 194 | 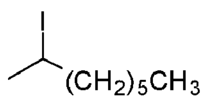 |
| 195 | 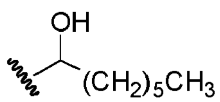 | 196 | 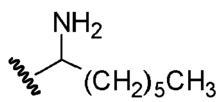 | 197 | 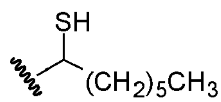 |
| 198 | 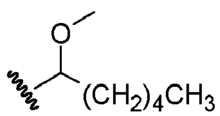 | 199 | 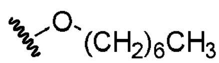 | 200 | 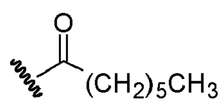 |
| 201 | 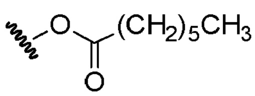 | 202 | 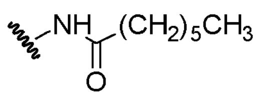 | 203 | 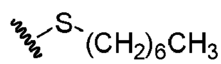 |

(continued)

| | | | | | |
|---|---|---|---|---|---|
| chain length = $\mathbf{C_7}$ | | | | | |
| 204 | 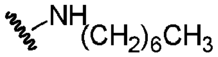 | 205 | $CH_2-(CH_2)_6OH$ | 206 | $CH_2-(CH_2)_6NH_2$ |
| 207 | $CH_2-(CH_2)_6SH$ | 208 | 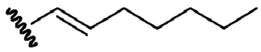 | 209 | 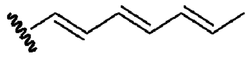 |
| 210 | 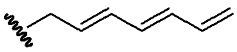 | 211 | 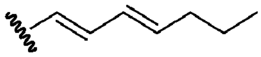 | 212 | 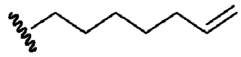 |
| 213 | 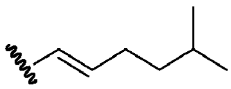 | 214 | 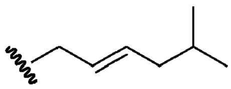 | 215 | 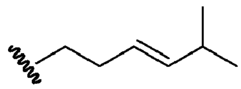 |
| 216 | 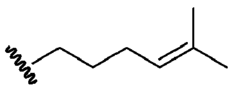 | 217 | 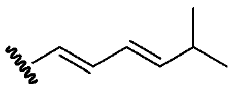 | 218 | 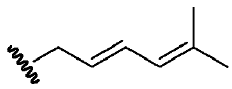 |
| 219 | 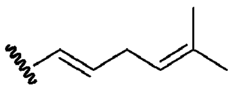 | | | | |
| chain length = $\mathbf{C_8}$ | | | | | |
| 220 | $(CH_2)_7CH_3$ | 221 | 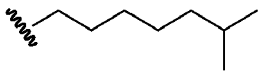 | 222 | 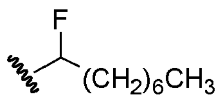 |
| 223 | 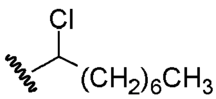 | 224 | 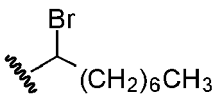 | 225 | 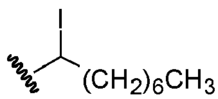 |
| 226 | 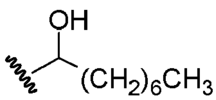 | 227 | 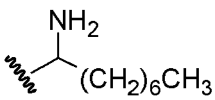 | 228 | 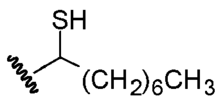 |
| 229 | 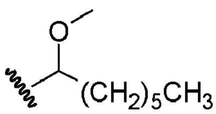 | 230 | 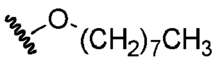 | 231 | 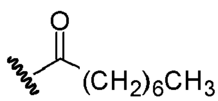 |
| 232 | 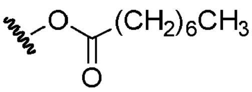 | 233 | 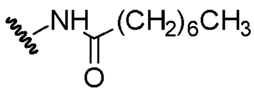 | 234 | 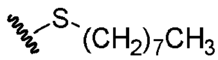 |
| 235 | 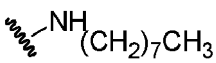 | 236 | $CH_2-(CH_2)_7OH$ | 237 | $CH_2-(CH_2)_7NH_2$ |
| 238 | $CH_2-(CH_2)_7SH$ | 239 | 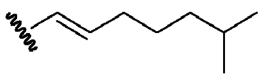 | 240 | 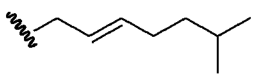 |
| 241 | 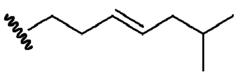 | 242 | 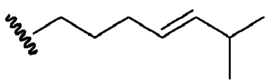 | 243 | 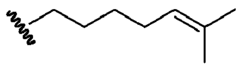 |
| 244 | 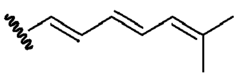 | 245 | 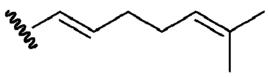 | 246 | 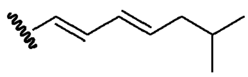 |

(continued)

| | | | | | |
|---|---|---|---|---|---|
| chain length = $C_8$ | | | | | |
| 247 | 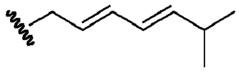 | 248 | 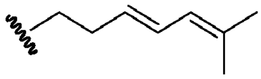 | | |
| chain length = $C_9$ | | | | | |
| 249 | 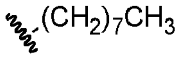 | 250 | 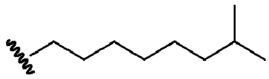 | 251 | 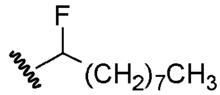 |
| 252 | 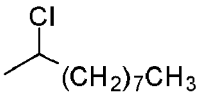 | 253 | 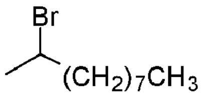 | 254 | 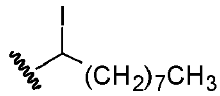 |
| 255 | 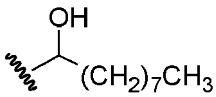 | 256 | 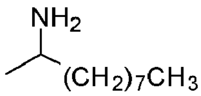 | 257 | 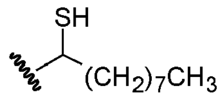 |
| 258 | 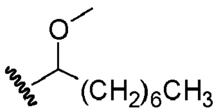 | 259 | 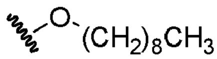 | 260 | 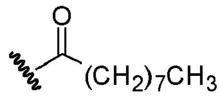 |
| 261 | 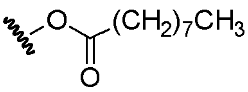 | 262 | 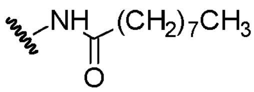 | 263 | 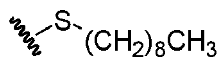 |
| 264 | 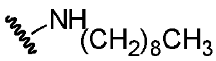 | 265 | 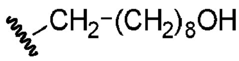 | 266 | 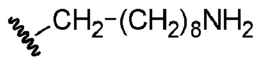 |
| 267 | 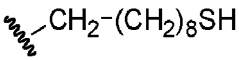 | 268 | 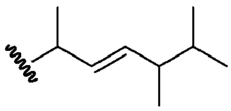 | 269 | 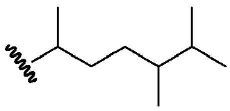 |
| chain length = $C_{10}$ | | | | | |
| 270 | 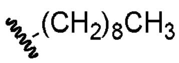 | 271 | 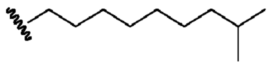 | 272 | 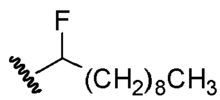 |
| 273 | 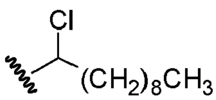 | 274 | 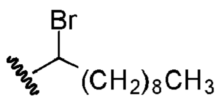 | 275 | 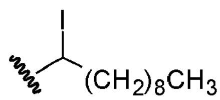 |
| 276 | 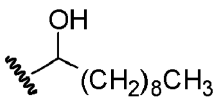 | 277 | 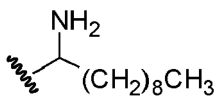 | 278 | 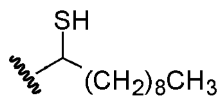 |
| 279 | 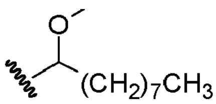 | 280 | 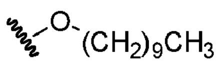 | 281 | 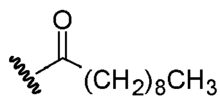 |
| 282 | 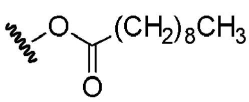 | 283 | 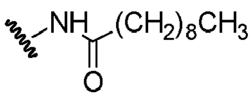 | 284 | 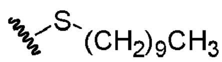 |

(continued)

| | | | | | |
|---|---|---|---|---|---|
| chain length = **$C_{10}$** | | | | | |
| 285 | 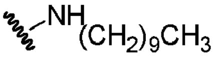 | 286 | 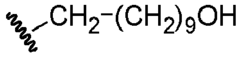 | 287 | 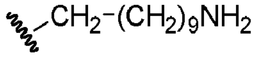 |
| 288 | 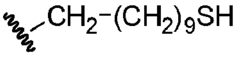 | 289 | 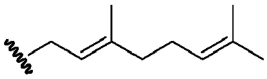 | 290 | 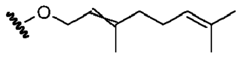 |
| 291 | 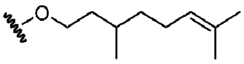 | 292 | 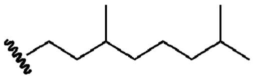 | 293 | 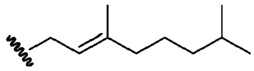 |
| 294 | 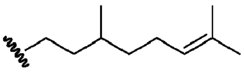 | | | | |
| chain length = **$C_{11}$** | | | | | |
| 295 | 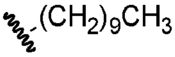 | 296 | 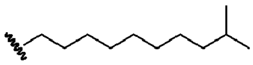 | 297 | 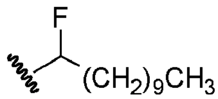 |
| 298 | 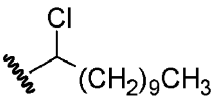 | 299 | 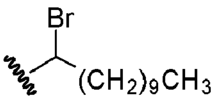 | 300 | 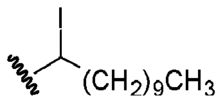 |
| 301 | 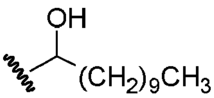 | 302 | 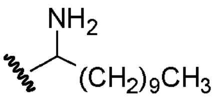 | 303 | 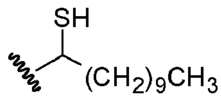 |
| 304 | 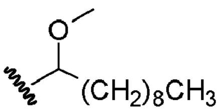 | 305 | 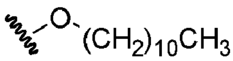 | 306 | 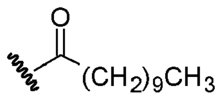 |
| 307 | 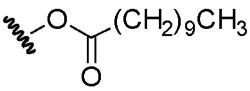 | 308 | 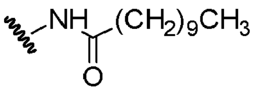 | 309 | 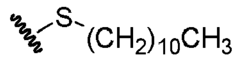 |
| 310 | 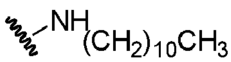 | 311 | 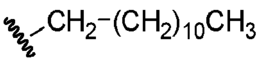 | 312 | 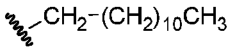 |
| 313 | 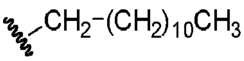 | | | | |
| chain length = **$C_{12}$** | | | | | |
| 314 | 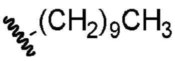 | 315 | 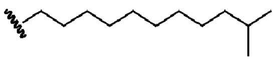 | 316 | 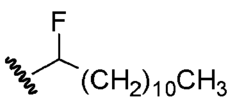 |
| 317 | 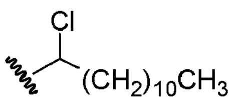 | 318 | 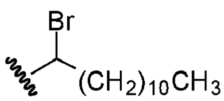 | 319 | 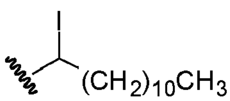 |
| 320 | 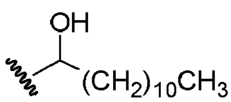 | 321 | 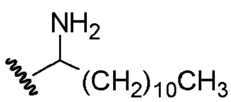 | 322 | 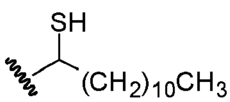 |

(continued)

| chain length = $\mathbf{C_{12}}$ | | | | | |
|---|---|---|---|---|---|
| 323 | 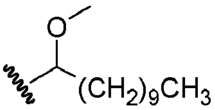 | 324 | 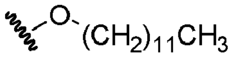 | 325 | 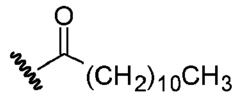 |
| 326 | 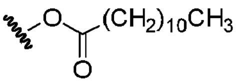 | 327 | 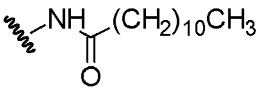 | 328 | 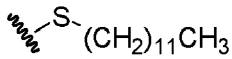 |
| 329 | 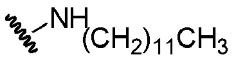 | 330 | 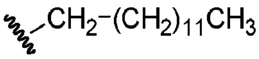 | 331 | 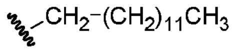 |
| 332 | 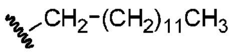 | | | | |
| chain length = $\mathbf{C_{14}}$ | | | | | |
| 333 | 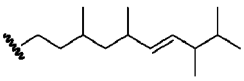 | 334 | 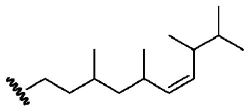 | 335 | 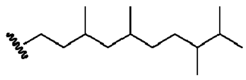 |
| chain length = $\mathbf{C_{15}}$ | | | | | |
| 336 | 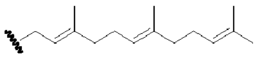 | 337 | 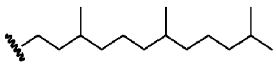 | | |
| chain = **Aryl** | | | | | |
| 338 | 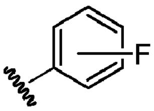 | 339 | 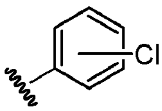 | 340 | 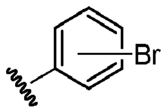 |
| 341 | 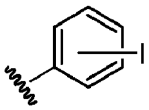 | 342 | 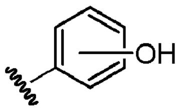 | 343 | 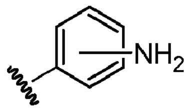 |
| 344 | 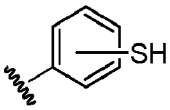 | 345 | 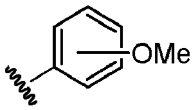 | 346 | 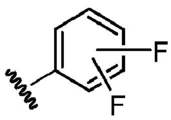 |
| 347 | 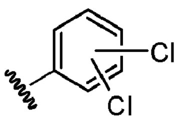 | 348 | 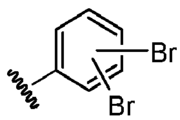 | 349 | 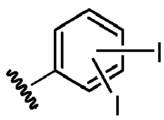 |
| 350 | 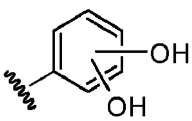 | 351 | 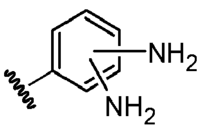 | 352 | 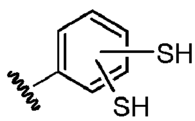 |
| 353 | 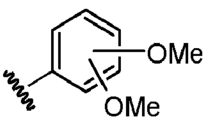 | 354 | 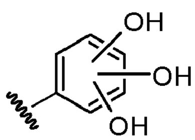 | 355 | 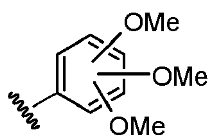 |

(continued)

| chain = **Aryl** | | | | | |
|---|---|---|---|---|---|
| 356 | 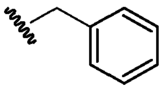 | 357 | 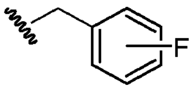 | 358 | 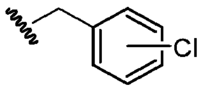 |
| 359 | 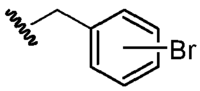 | 360 | 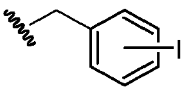 | 361 | 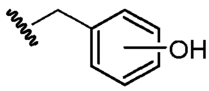 |
| 362 | 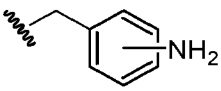 | 363 | 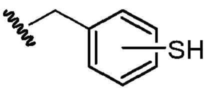 | 364 | 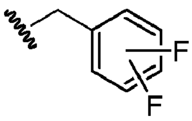 |
| 365 | 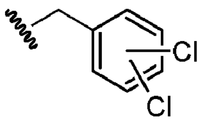 | 366 | 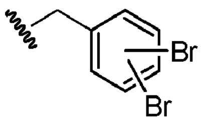 | 367 | 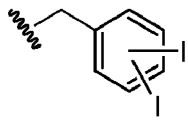 |
| 368 | 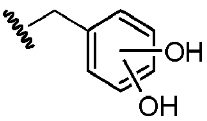 | 369 | 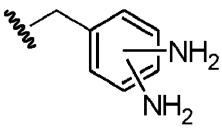 | 370 | 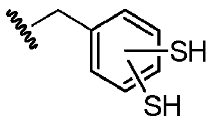 |
| 371 | 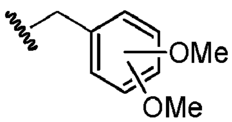 | 372 | 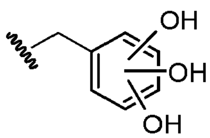 | 373 | 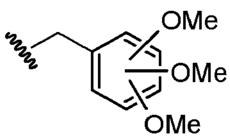 |
| 374 | 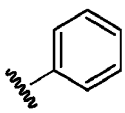 | | | | |

Optionally, at least one of R, R1, R2, R3 and R4 is substituted with a halogen, e.g. Cl, I, or Br, preferably I.

Preferably, one or both of X and Q are OH, SH, or NHR, each the same or different, as it is at present assumed that a hydrogen bridge from the to the neighbouring keto group, e.g. shown as position C4 or C1 in structure II, in combination with the naphthazarine basic framework supports that the absorption wavelength is in the range of 450 to 700 nm, e.g. 500 to 600 nm, which can be irradiated through nail or hooves.

An embodiment of the pharmaceutical compound of structure I has structure II

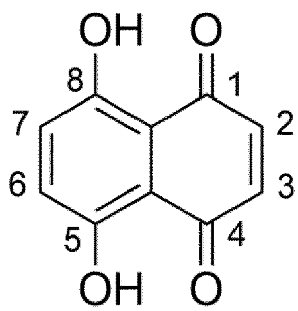

(structure II),

wherein C2 is substituted with R1,

C3 is substituted with R2,

C5 is substituted with Q, which is e.g. OH as shown in structure II, or NHR or SH,
C8 is substituted with X, which is e.g. OH, as shown in structure II, or NHR or SH,
C6 is substituted with R3, e.g. a halogen, or H,
C7 is substituted with R4, e.g. a halogen, or H,
optionally only one of C6 and C7 is substituted with a halogen or both of C6 and C7 are substituted with H, and the other C-atoms are substituted with H. Structure II shows the naphthazarin basic framework.

Optionally, in structure II C2 is substituted with R1, e.g. selected from Table 1, and each of C3, C6 and C7 are substituted with hydrogen.

It was found that the wavelength that is absorbed by the pharmaceutical compound, as well as the migration of the compound into nail or hoof, and/or preferably the metabolic stability of the compound can be varied by the length and degree of saturation of the hydrocarbon group forming R1 and R2, and by its substitution with a halogen atom.

Especially in respect of compounds of Structure I in which at least one of R3 and R4 is a halogen and each of Z and Y is C, and/or at least one of R, R1, R2, R3 and R4 is substituted with a halogen, the invention relates to the compounds for use in medical treatment, the treatment preferably comprising photodynamic therapy, e.g. irradiation of the compound after its topical administration.

Further, it has been found that substitution of one or both of Z and Y, e.g. of at least one of C6 and C7, by a halogen, the halogen forming at least one of R3 and R4, is suitable for adjusting the wavelength that is absorbed by the pharmaceutical compound, as well as the migration of the compound into nail or hoof, as well as preferably the metabolic stability of the compound.

The compounds of the invention have the advantage of generating ROS under irradiation at 450 to 700 nm, e.g. 515 ± 20 nm, preferably 515 ± 10 nm, which is a wavelength for which nail or hoof is sufficiently transmissible. Accordingly, these pharmaceutical compounds are suitable for use in the treatment of onychomycoses as photodynamic treatment, also referred to as photodynamic therapy, by irradiation is effective in generating ROS in those regions within the nail or hoof in which the pharmaceutical compound is present. Preferably, subsequent to topical application of the compound to a nail or hoof, the treatment comprises the step of removing the compound from skin and/or covering skin surrounding the nail to which the compound has been applied topically with a material that is not permissive to the irradiation applied to the nail. Removing the compound from skin and/or covering the skin to which the compound may have been applied is preferred for preventing irradiation, e.g. of 450 to 700 nm, applied to the nail and ROS generated by the compound to affect the skin.

Further, the invention relates to a process for producing the compound for use in the treatment of onychomycoses, and to use of the compound for producing a pharmaceutic composition for treatment of onychomycoses, wherein the treatment is photodynamic treatment, preferably comprising irradiation of the compound following its application to a nail or hoof.

The following table shows specific pharmaceutical compounds of the invention and their IC50 values, upper value without irradiation, lower value with irradiation, as determined in in vitro assays.

| name | structure | Ca [nM] IC50 without irradiation IC50 with irradiation | Tru [nM] IC50 without irradiation IC50 with irradiation |
|---|---|---|---|
| Naphthazarin | 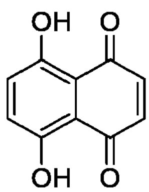 | 2219 ± 511<br>632 ± 175<br><br>(n=4) | 2476 ± 1506<br>353 ± 237<br><br>(n=5) |
| BJ-18 | 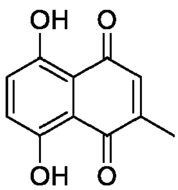 | 10933 ± 4079<br>2337 ± 846<br><br>(n=3) | -<br>87.6 ± 65.4<br><br>(n=2) |

(continued)

| name | structure | Ca [nM] IC50 without irradiation IC50 with irradiation | Tru [nM] IC50 without irradiation IC50 with irradiation |
|---|---|---|---|
| BJ-34 | 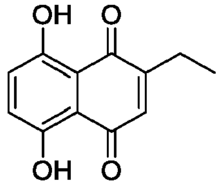 | -<br>715 ± 209<br>(n=3) | -<br>24.2 ± 20.6<br>(n=4) |
| BJ-35 | 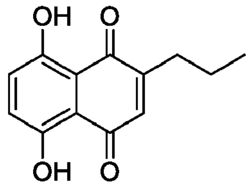 | -<br>599 ± 289<br>(n=3) | -<br>13.0 ± 12.2<br>(n=3) |
| BJ-36 | 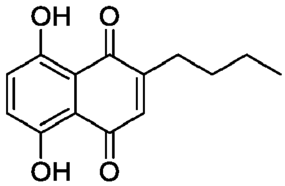 | -<br>556 ± 355<br>(n=3) | -<br>1.0 ± 0.3<br>(n=3) |
| DOS | 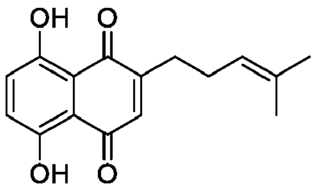 | -<br>213 ± 55<br>(n=5) | -<br>32.2 ± 17.4<br>(n=5) |
| BJ-28 | 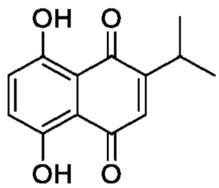 | -<br>1042 ± 185<br>(n=2) | -<br>42.7<br>(n=1) |
| BJ-19 | 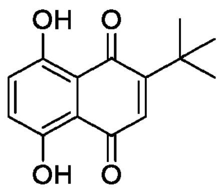 | -<br>7646 ± 4479<br>(n=2) | -<br>244 ± 192<br>(n=3) |
| BJ-27 | 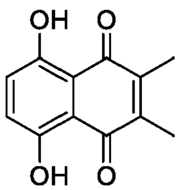 | -<br>2746 ± 70<br>(n=2) | -<br>321 ± 212<br>(n=2) |
| BJ-39 | 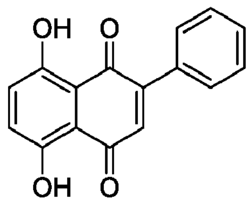 | nd | -<br>2.9<br>(n=1) |

(continued)

| name | structure | Ca [nM] IC50 without irradiation IC50 with irradiation | Tru [nM] IC50 without irradiation IC50 with irradiation |
|---|---|---|---|
| BJ-49 | 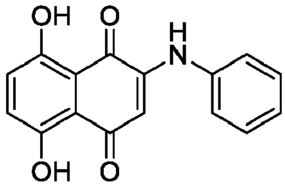 | -<br>570<br><br>(n=1) | -<br>352 ± 150<br><br>(n=2) |
| BJ-SK-Amin | 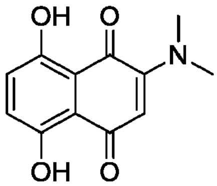 | -<br>1484<br><br>(n=1) | 8142<br>3338<br><br>(n=1) |
| Shikonin/ Alkannin | 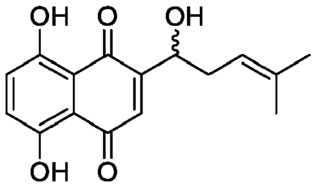 | 1484<br><br>(n=1) | 10510 ± 6917<br>781 ± 407<br><br>(n=3) |
| Clotrimazol | Comparative compound for Tru | nd | 114 ± 61<br>109 ± 51<br>(n=10) |
| Nystatin | Comparative compound for Ca | 692 ± 128<br>1665 ± 886<br>(n=7) | nd |

wherein the curve fit was determined using Graphpad Prism 7 by the equation log(inhibitor) vs. response - variable slope, and

$$Y=Bottom + (Top-Bottom)/(1+10^{((LogIC50-X)*HillSlope)}),$$

with Top and Bottom determined as the plateaus of the Y-axis values, HillSlope describing the steepness of curves, with HillSlope = -1 as a suitable standard,
X = Log of active compound concentration, Y = value of response.
Ca = antimycotic activity against Candida albicans
Tru = antimycotic activity against Trichophyton rubrum
nd = not determined
\- = no fit possible

For comparison, 2-hydroxy-1,4-naphthochinone, which is a component of henna dye, was tested, and showed essentially no antimycotic effect upon irradiation.

The invention is now described in greater detail by way of examples and with reference to the figures which show in

- Fig. 1A and 1B the inhibition of C. albicans and of T. rubrum by exemplary pharmaceutical compounds,
- Fig. 2 a micrograph of a cross-section of a keratin plate with indication of measurement depth, and in
- Fig. 3 analyses of the migration of comparative compound Amorolfin in Fig. 3A, of comparative compound Ciclopirox in Fig. 3B, and of Deoxyshikonin according to the invention in Fig. 3C.

Example 1: Anti-mycotic activity assay in pathogen suspension

For determining the IC50 of the anti-mycotic activity of compounds, the human pathogen Candida albicans, strain DSM 1386, or clinical isolates of Trichophyton rubrum obtained from patient samples were used.

Candida albicans DSM 1386 was cultivated overnight in medium M186/3 (3.3% glucose, 0.16% peptone from soybeans, 0.1% yeast extract, 0.1% malt extract). Test samples were added in triplicates to a 96-well plate for a final assay concentration of 10μM and 100μM of pharmaceutical compounds. 200 μl of the yeast was added to each well after diluting the overnight culture to an optical density (600 nm) of 0.03. The wells were irradiated using a custom-made light source with a peak wavelength of 519 nm (8 LEDs, 4600 mW, 6.5 mW/cm$^2$, Fa. Sahlmann Photochemical Solutions, Bad Segeberg, Germany). Handling of the test compounds and well plates took place in a darkened room equipped with red-light LEDs to protect them from green light. Microplates were incubated for 5 h at 28 °C on a shaker at 200 rpm. Subsequently, 10 μl resazurin solution (0.3 mg/ml in phosphate buffer) was added to each well and the microplates were incubated again for 30 min before measuring fluorescence (560/590 nm) using a microplate reader (Tecan Infinite M200, Tecan, Männedorf, Switzerland). Nystatin was used as positive control. For IC50 determination a dilution series of the test samples was prepared and IC50 value was calculated by Graph Pad Prism 7 as the concentration that show 50% inhibition of growing.

Trichophyton rubrum I/95 (patient isolate) was cultivated for 1 week on GPY (0.1% glucose, 0.05% petone, 0.01 yeast extract, 1.5% agar) at 28°C. A suspension of $5 \times 10^4$ spores/ml in M186 (1% glucose, 0.5% peptone from soybeans, 0.3% yeast extract, 0.3% malt extract) was prepared and added to each well of a microplate containing one of the pharmaceutical compounds, which were pipetted in triplicates for a final concentration of 10μm and 100μM. The wells were irradiated using the custom-made light source with a peak wavelength of 519 nm. Handling of the test compounds and well plates took place in a darkened room equipped with a red-light LED to protect them from green light. After incubation for 72h at 28°C and 120rpm the optical density at 600nm was measured using the microplate reader Tecan Infinite M200. The resulting values were compared with a positive control (10μM Clotrimazol) and a negative control (no compound) on the same plate. For IC50 determination a dilution series of the test samples was prepared and IC50 value was calculated by Graph Pad Prism 7 as the concentration that show 50% inhibition of growing.

Fig. 1A shows the IC50 values for deoxyshikonin against Candida albicans strain and Fig. 1B against the T. rubrum patient isolate, both in the absence of activating irradiation (lower curves, dark) and in the presence of activating irradiation (519 nm, upper curves). The IC50 under irradiation against C. albicans was determined to 213.3 ± 54.7 nM, against T. rubrum to 32.2 ± 17.4 nM. For the comparative compound Nystatin an IC50 = 692.4 ± 140.4 nM without irradiation, and a lower activity under irradiation, which is believed to be caused by decomposition of Nystatin under irradiation.

The results are summarized in the above table, showing that the pharmaceutical compounds of the invention under irradiation are effective against the pathogens causing onychomycoses. Further, the results show that the activity of the pharmaceutical compounds is significantly lower in the absence of irradiation, which allows application of the compounds in the absence of activating irradiation without generating ROS that might damage soft tissue.

Example 2: Permeation of pharmaceutical compounds into nail

For testing the permeation of pharmaceutical compounds into nail, a Franz diffusion cell (obtained from Permegear, Germany) was used, in which a bovine hoof plate, 2 mm thickness, is arranged to separate a first volume of phosphate buffered saline pH = 8.1 with 42 % vol./vol. ethanol containing a pharmaceutical compound from a second volume of the same fluid but without pharmaceutical compound, wherein the second volume is circulated for 20 h at 20 °C and then analysed for presence of the pharmaceutical compound. It was found that the compounds of the invention migrated from the first volume into the hoof plate, and the compounds could not be detected in the second volume. This indicates that the pharmaceutical compounds migrate into the keratin plate that was represented by the hoof plate, and that the compounds essentially did not diffuse out of the keratin plate into the second volume. This shows that the compounds of the invention permeate into the keratin and do not diffuse out of the keratin, indicating that nail matrix adjacent to the keratin does not receive the compounds, and therefore the nail matrix is not directly affected by activity of the pharmaceutical compounds under irradiation.

As a further analysis, a bovine hoof plate, 2 mm thickness, was arranged on water-saturated cotton gauze, and pharmaceutical compound was deposited on the opposite surface of the hoof plate. This arrangement was incubated under static conditions at room temperature for 5 days. The pharmaceutical compound was used at 5 wt.-% in a preliminary formulation of 12.5 wt.-% Eudragit RL PO, 60 wt.-% ethanol, 15 wt.-% ethyl acetate, 5 wt.-% butyl acetate, 2.5 wt.-% triacetin, which formulation should mimic the Loceryl lacquer. For comparison, the topic antimycotics Amorolfin in Loceryl lacquer and Ciclopirox in Ciclopoli were used in parallel.

After the incubation for 5 d, the lacquer compositions were carefully removed and cross-sections from the hoof plates were made. Fig. 2 shows a microscopic picture of a hoof plate cross-section, with an indication of measurement

points X numbered 1.0, 1.1, 1.2 close to the surface, and measurement points X numbered 2.0, 2.1, 2.2 at a depth of 10 μm. This pattern of measurement points, each at a depth greater by 10 μm, up to a depth of at least 100 μm, was used for Raman spectroscopy for presence of pharmaceutical compound.

Fig. 3A for Amorolfin, Fig. 3B for Ciclopirox, and Fig. 3C for Deoxyshikonin as a representative of compounds of the invention, shows the concentration of the respective compound in the different depths of the hoof plate, wherein increasing depths showed lower compound concentrations. Raman spectroscopy showed that the comparative Amorolfin could not be detected inside the keratin cross-section as only the keratin baseline was detected. For Ciclopirox, migration up to a depth of 40 μm into the keratin cross-section was found. Deoxyshikonin was found to migrate to a depth of more than 50 μm into the keratin cross-section.

On the exemplary pharmaceutical compound of the invention, these results show that the compounds of the invention show good penetration into nails and hooves, even when using it in the preliminary lacquer formulation that was not optimized, with only one topical application.

<u>Example 3: Anti-mycotic activity assay from nail</u>

For testing the effect of the pharmaceutical compounds in a nail, compounds were applied as a solution of 5 mg/ml or 10 mg/ml in nitrocellulose-based cosmetic nail varnish, diluted 1:1 with ethyl acetated, onto cow horn membranes representing nail.

Trichophyton rubrum I/95 (patient isolates) was cultivated for 1-3 weeks on GPY (0.1% glucose, 0.05% peptone, 0.01 yeast extract, 1.5% agar) at 28°C. For the infected nail model, petri dishes with M186 agar (1% glucose, 0.5% peptone from soybeans, 0.3% yeast extract, 0.3% malt extract, 1.5% agar, supplemented with 100 U/ml penicillin and 100mg/ml streptomycin) where inoculated with the pathogen using sterile cotton swabs. Afterwards autoclaved cow horn membranes (in DPBS-buffer solution, 100 μm thick, 8 mm in diameter) where put onto the agar (5 per petri dish) followed by incubation for 48 h at 29 °C. As an example of the invention, Deoxyshikonin was used, and Clotrimazol as control, the diluted nail varnish as vehicle control. The formulations were applied to 15 μL/membrane to the cow horn membranes using an Eppendorf pipette. After incubation for another 24 h four membranes of each type (control, vehicle, 5 mg/ml or 10 mg/ml Deoxyshikonin) were transferred onto fresh petri dishes with the surface to which the formulation was applied now facing the agar. The dishes were irradiated two times for 5 min each with a peak wavelength of 519 nm (8 LEDs, 4600 mW, 6.5 $mW/cm^2$). Afterwards formulations were applied (15 μL/membrane) onto the untreated side of the membranes (facing upwards), they were allowed to dry and were irradiated again for 5 min (2x). The dishes where incubated for 24 h at 29 °C and irradiated again for 5 min (2x). After 7 days of incubation at 29 °C the dishes were evaluated. Anytime handling the compounds and the agar plates after application of the formulations the ambient light was turned off and a red-light LED was used to protect them from any activating light source.

It was found that with irradiation the Deoxyshikonin from the cow horn membranes effectively suppressed growth of T. rubrum on and in the horn membranes as well as in the surrounding medium, whereas without irradiation, growth of T. rubrum was reduced compared to both controls but not suppressed.

This result shows that the compounds of the invention are effective in the treatment of onychomycoses in photodynamic treatment using irradiation of the nail to which the compound has been applied, wherein the irradiation has a wavelength for which nail is permeable and which wavelength activates the compound. A preferred wavelength is about 515 nm.

**Claims**

1. Pharmaceutical compound for use in the treatment of onychomycoses, the treatment comprising irradiation of the nail at a wavelength of 450 to 700 nm, **characterized in that** the compound comprises structure I

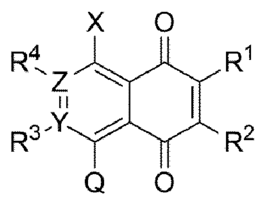

(Structure I),

wherein Z is one of C and N, Y is one of C and N, X is OH or SH or NHR or $NR_2$, Q is OH or SH or NHR or $NR_2$, preferably X is OH and Q is OH, and each of R, R1, R2, R3 and R4 is independently a $C_1$ to $C_{12}$ linear, branched or cyclic alkyl or aryl, or a halogen.

**2.** Pharmaceutical compound for use in the treatment of onychomycoses according to claim 1, comprising structure II

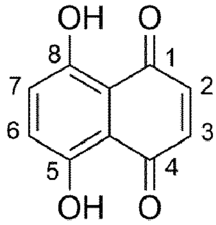 (structure II),

wherein C2 is substituted with R1, or C2 and C3 are substituted with the same or a different R1.

**3.** Pharmaceutical compound for use in the treatment of onychomycoses according to claim 2, wherein at least one of C6 and C7 is substituted with a halogen.

**4.** Pharmaceutical compound for use in the treatment of onychomycoses according to one of the preceding claims, wherein at least one of R, R1, R2, R3 and R4 is substituted with a halogen atom.

**5.** Pharmaceutical compound for use in the treatment of onychomycoses according to one of the preceding claims, wherein each of R, R1, R2, R3 and R4 is hydrogen or one of

| number | structure | number | structure | number | structure |
|---|---|---|---|---|---|
| 1 | 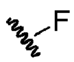 | 2 | 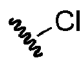 | 3 | 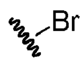 |
| 4 | 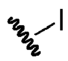 | 5 | 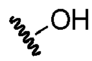 | 6 | 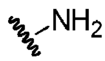 |
| 7 | 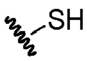 | 8 | 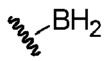 | | |
| $C_1$ | | | | | |
| 9 | 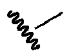 | 10 | 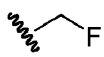 | 11 | 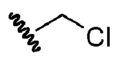 |
| 12 | 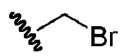 | 13 | 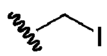 | 14 | 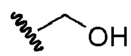 |
| 15 | 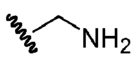 | 16 | 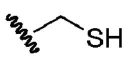 | 17 | 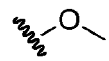 |
| 18 | 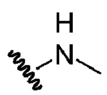 | 19 | 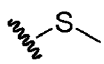 | 20 | 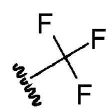 |
| 21 | 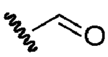 | | | | |
| $C_2$ | | | | | |
| 22 | 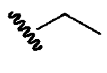 | 23 | 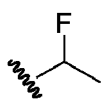 | 24 | 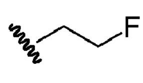 |

(continued)

| number | structure | number | structure | number | structure |
|---|---|---|---|---|---|
| 25 | 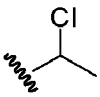 | 26 | 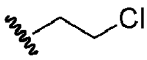 | 27 | 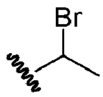 |
| 28 | 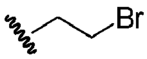 | 29 | 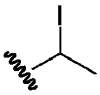 | 30 | 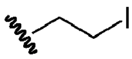 |
| 31 | 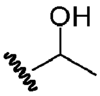 | 32 | 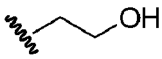 | 33 | 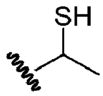 |
| 34 | 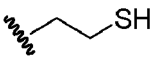 | 35 | 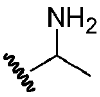 | 36 | 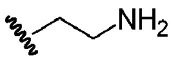 |
| 37 | 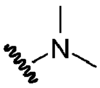 | 38 | 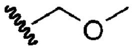 | 39 | 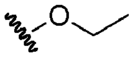 |
| 40 | 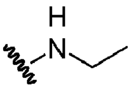 | 41 | 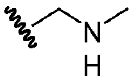 | 42 | 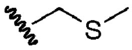 |
| 43 | 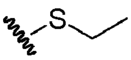 | 44 | 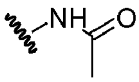 | 45 | 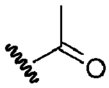 |
| 46 | 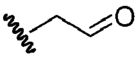 | 47 | 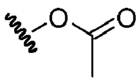 | | |
| **$C_3$** | | | | | |
| 48 | 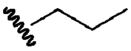 | 49 | 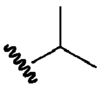 | 50 | 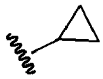 |
| 51 | 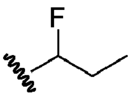 | 52 | 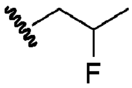 | 53 | 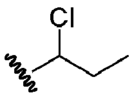 |
| 54 | 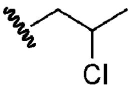 | 55 | 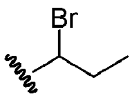 | 56 | 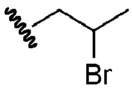 |
| 57 | 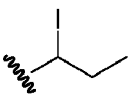 | 58 | 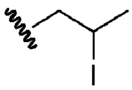 | 59 | 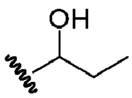 |
| 60 | 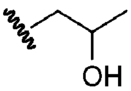 | 61 | 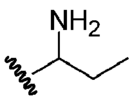 | 62 | 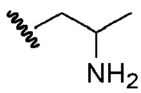 |
| 63 | 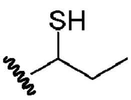 | 64 | 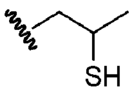 | 65 | |

(continued)

| number | structure | number | structure | number | structure |
|---|---|---|---|---|---|
| 66 | 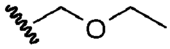 | 67 | 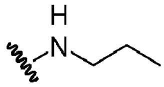 | 68 | 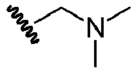 |
| 69 | 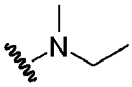 | 70 | 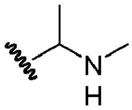 | 71 | 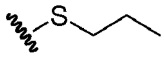 |
| 72 | 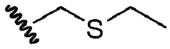 | 73 | 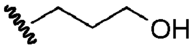 | 74 | 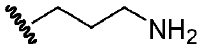 |
| 75 | 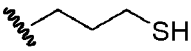 | 76 | 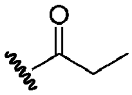 | 77 | 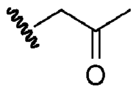 |
| 78 | 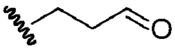 | 79 | 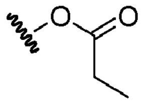 | 80 | 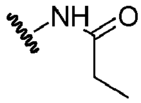 |
| 81 | 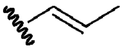 | 82 | 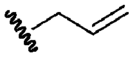 | | |
| $C_4$ | | | | | |
| 83 | 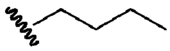 | 84 | 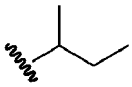 | 85 | 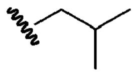 |
| 86 | 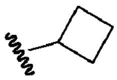 | 87 | 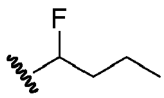 | 88 | 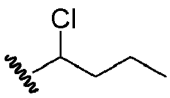 |
| 89 | 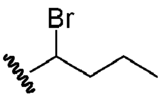 | 90 | 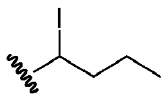 | 91 | 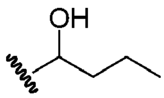 |
| 92 | 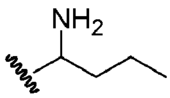 | 93 | 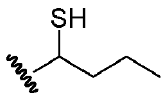 | 94 | 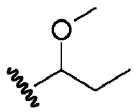 |
| 95 | 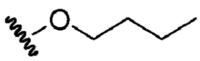 | 96 | 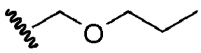 | 97 | 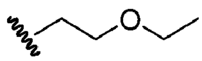 |
| 98 | 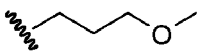 | 99 | 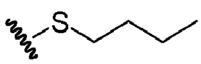 | 100 | 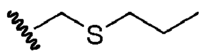 |
| 101 | 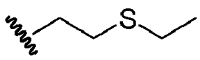 | 102 | 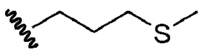 | 103 | 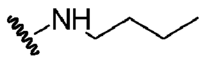 |
| 104 | 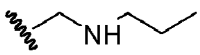 | 105 | 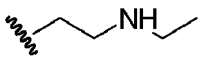 | 106 | |
| 107 | | 108 | | 109 | |

(continued)

| number | structure | number | structure | number | structure |
|---|---|---|---|---|---|
| 110 | 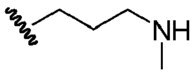 | 111 | 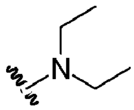 | 112 | 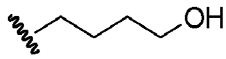 |
| 113 | 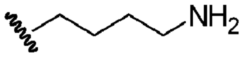 | 114 | 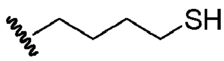 | 115 | 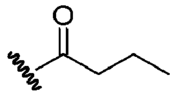 |
| 116 | 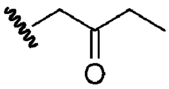 | 117 | 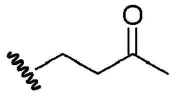 | 118 | 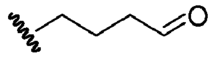 |
| 119 | 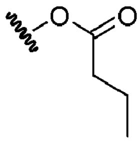 | 120 | 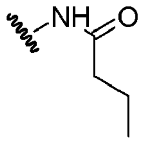 | 121 | 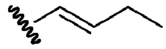 |
| 122 | 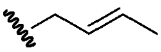 | 123 | 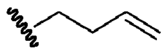 | 124 | 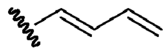 |
| **$C_5$** | | | | | |
| 125 | 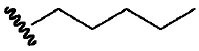 | 126 | 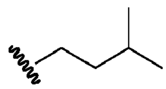 | 127 | 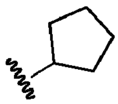 |
| 128 | 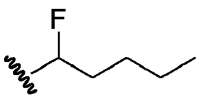 | 129 | 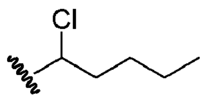 | 130 | 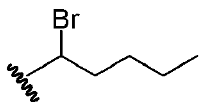 |
| 131 | 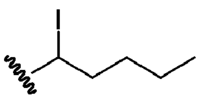 | 132 | 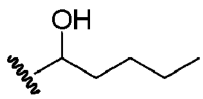 | 133 | 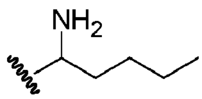 |
| 134 | 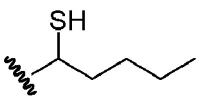 | 135 | 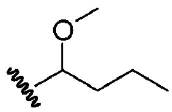 | 136 | 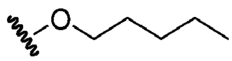 |
| 137 | 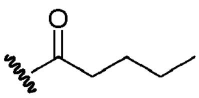 | 138 | 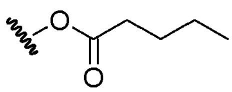 | 139 | 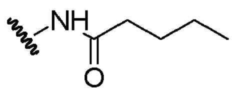 |
| 140 | 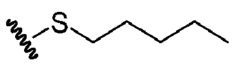 | 141 | 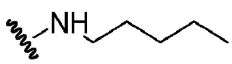 | 142 | 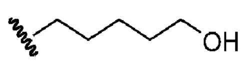 |
| 143 | 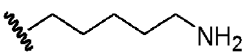 | 144 | 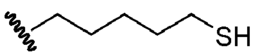 | 145 | 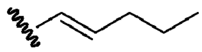 |
| 146 | 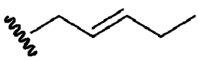 | 147 | 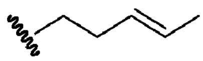 | 148 | 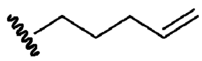 |
| 149 | 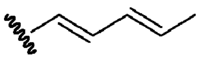 | 150 | | 151 | |

(continued)

| number | structure | number | structure | number | structure |
|---|---|---|---|---|---|
| 152 | 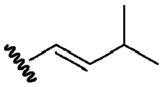 | 153 | 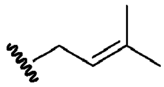 | 154 | 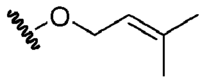 |
| | | | $C_6$ | | |
| 155 | 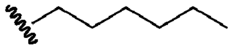 | 156 | 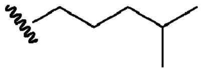 | 157 | 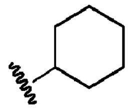 |
| 158 | 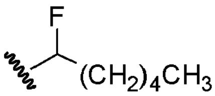 | 159 | 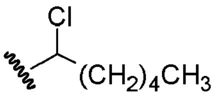 | 160 | 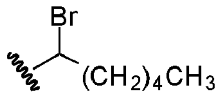 |
| 161 | 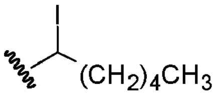 | 162 | 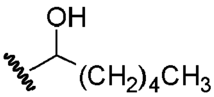 | 163 | 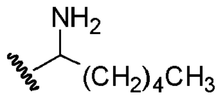 |
| 164 | 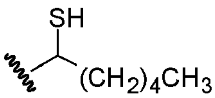 | 165 | 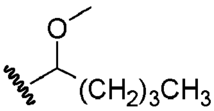 | 166 | 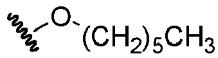 |
| 167 | 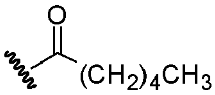 | 168 | 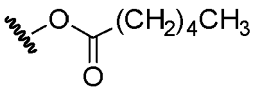 | 169 | 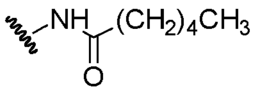 |
| 170 | 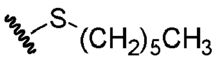 | 171 | 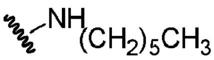 | 172 | $CH_2-(CH_2)_5OH$ |
| 173 | $CH_2-(CH_2)_5NH_2$ | 174 | $CH_2-(CH_2)_5SH$ | 175 | 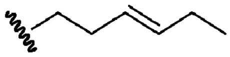 |
| 176 | 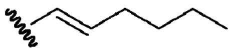 | 177 | 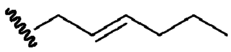 | 178 | 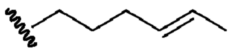 |
| 179 | 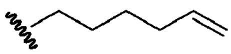 | 180 | 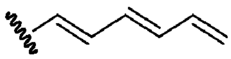 | 181 | 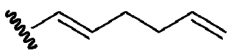 |
| 182 | 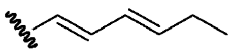 | 183 | 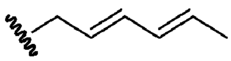 | 184 | 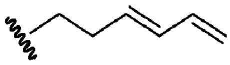 |
| 185 | 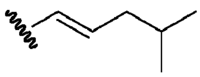 | 186 | 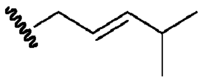 | 187 | 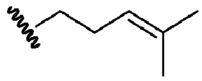 |
| 188 | 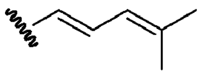 | | | | |
| | | | $C_7$ | | |
| 189 | 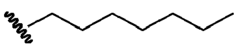 | 190 | 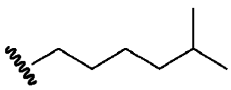 | 191 | 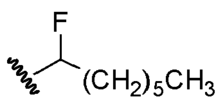 |
| 192 | 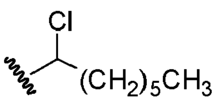 | 193 | 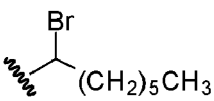 | 194 | 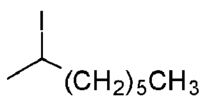 |

(continued)

| number | structure | number | structure | number | structure |
|---|---|---|---|---|---|
| 195 | 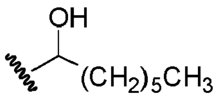 | 196 | 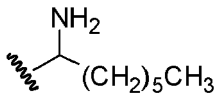 | 197 | 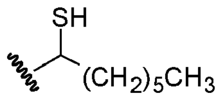 |
| 198 | 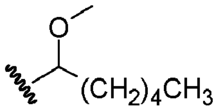 | 199 | 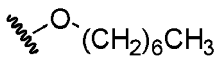 | 200 | 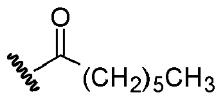 |
| 201 | 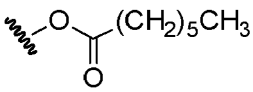 | 202 | 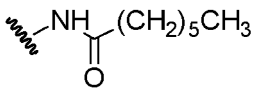 | 203 | 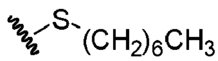 |
| 204 | 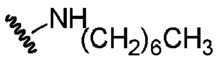 | 205 | $CH_2-(CH_2)_6OH$ | 206 | $CH_2-(CH_2)_6NH_2$ |
| 207 | $CH_2-(CH_2)_6SH$ | 208 | 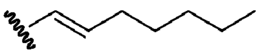 | 209 | 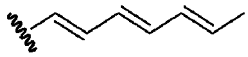 |
| 210 | 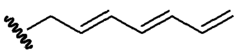 | 211 | 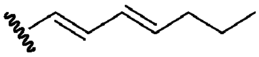 | 212 | 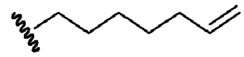 |
| 213 | 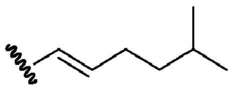 | 214 | 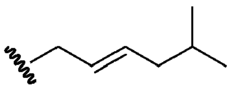 | 215 | 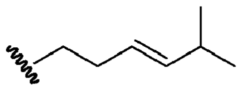 |
| 216 | 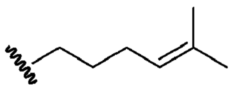 | 217 | 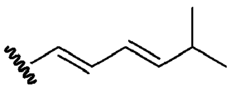 | 218 | 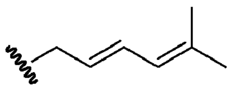 |
| 219 | 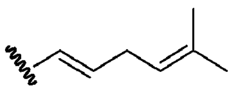 | | | | |
| **$C_8$** | | | | | |
| 220 | $(CH_2)_7CH_3$ | 221 | 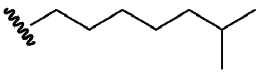 | 222 | 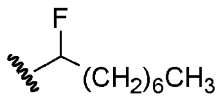 |
| 223 | 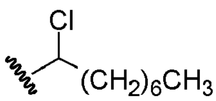 | 224 | 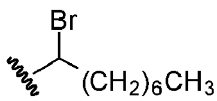 | 225 | 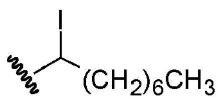 |
| 226 | 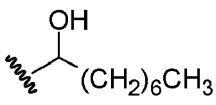 | 227 | 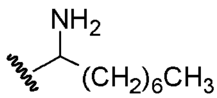 | 228 | 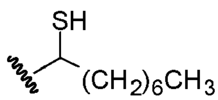 |
| 229 | 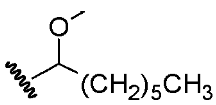 | 230 | 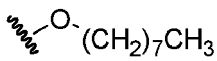 | 231 | 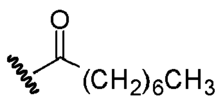 |
| 232 | 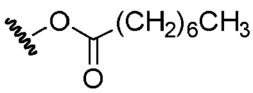 | 233 | 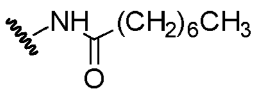 | 234 | 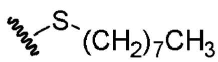 |
| 235 | 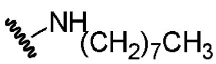 | 236 | $CH_2-(CH_2)_7OH$ | 237 | $CH_2-(CH_2)_7NH_2$ |

(continued)

| number | structure | number | structure | number | structure |
|---|---|---|---|---|---|
| 238 | 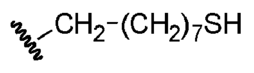 | 239 | 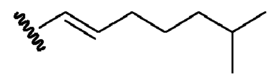 | 240 | 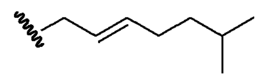 |
| 241 | 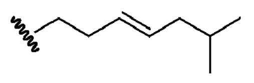 | 242 | 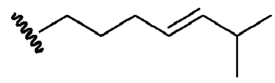 | 243 | 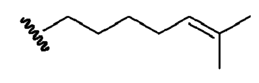 |
| 244 | 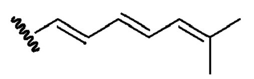 | 245 | 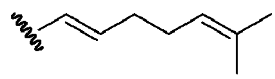 | 246 | 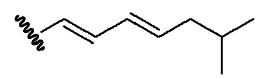 |
| 247 | 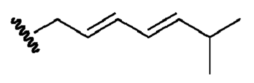 | 248 | 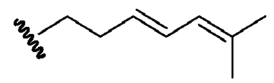 | | |
| | | **$C_9$** | | | |
| 249 | 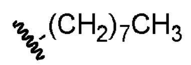 | 250 | 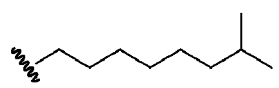 | 251 | 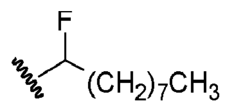 |
| 252 | 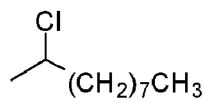 | 253 | 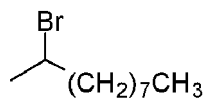 | 254 | 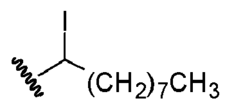 |
| 255 | 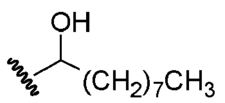 | 256 | 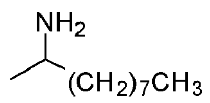 | 257 | 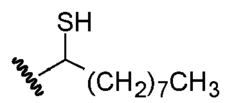 |
| 258 | 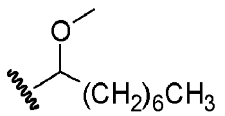 | 259 | 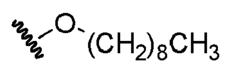 | 260 | 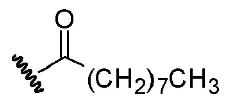 |
| 261 | 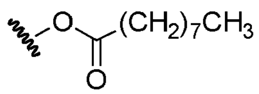 | 262 | 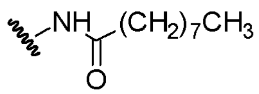 | 263 | 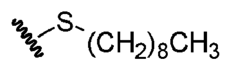 |
| 264 | 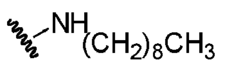 | 265 | 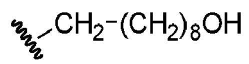 | 266 | 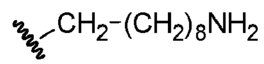 |
| 267 | 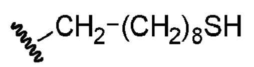 | 268 | 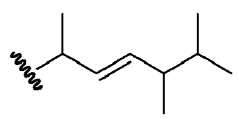 | 269 | 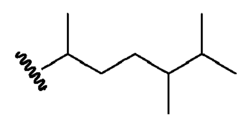 |
| | | **$C_{10}$** | | | |
| 270 | 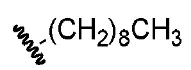 | 271 | 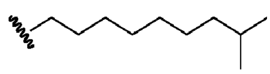 | 272 | 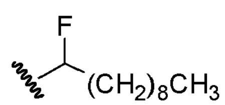 |
| 273 | 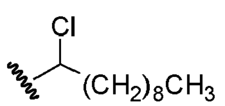 | 274 | 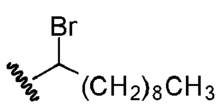 | 275 | 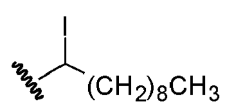 |
| 276 | 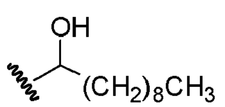 | 277 | 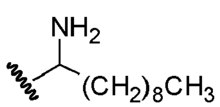 | 278 | |

(continued)

| number | structure | number | structure | number | structure |
|---|---|---|---|---|---|
| 279 | 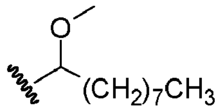 | 280 | 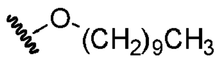 | 281 | 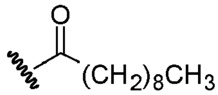 |
| 282 | 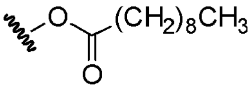 | 283 | 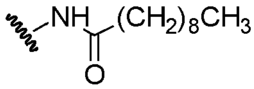 | 284 | 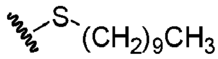 |
| 285 | 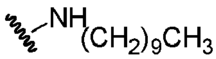 | 286 | 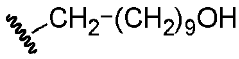 | 287 | 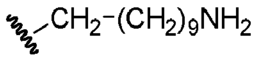 |
| 288 | 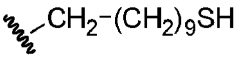 | 289 | 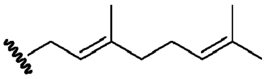 | 290 | 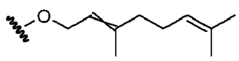 |
| 291 | 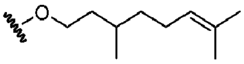 | 292 | 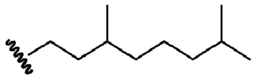 | 293 | 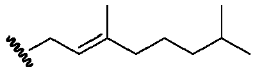 |
| 294 | 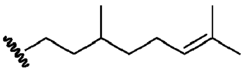 | | | | |
| **$C_{11}$** | | | | | |
| 295 | 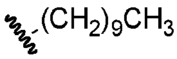 | 296 | 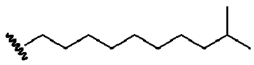 | 297 | 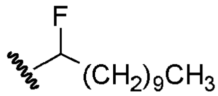 |
| 298 | 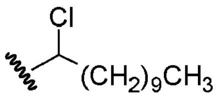 | 299 | 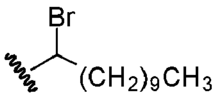 | 300 | 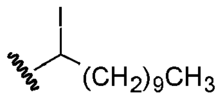 |
| 301 | 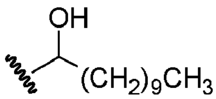 | 302 | 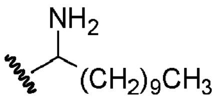 | 303 | 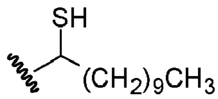 |
| 304 | 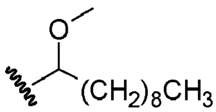 | 305 | 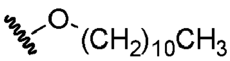 | 306 | 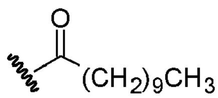 |
| 307 | 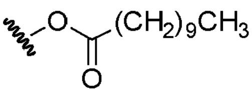 | 308 | 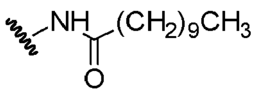 | 309 | 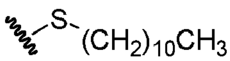 |
| 310 | 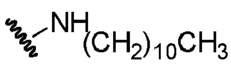 | 311 | 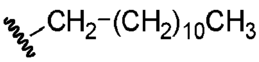 | 312 | 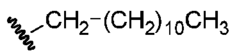 |
| 313 | 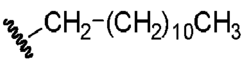 | | | | |
| **$C_{12}$** | | | | | |
| 314 | 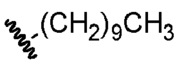 | 315 | 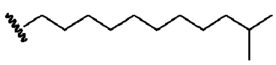 | 316 | 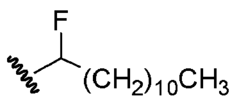 |

(continued)

| number | structure | number | structure | number | structure |
|---|---|---|---|---|---|
| 317 | 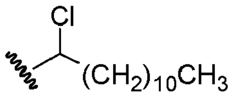 | 318 | 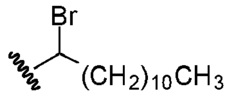 | 319 | 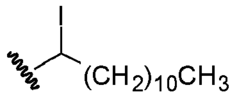 |
| 320 | 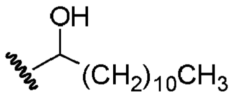 | 321 | 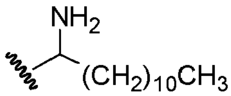 | 322 | 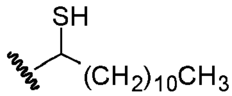 |
| 323 | 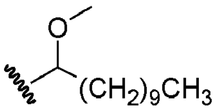 | 324 | 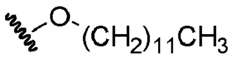 | 325 | 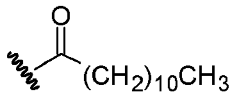 |
| 326 | 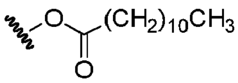 | 327 | 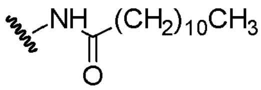 | 328 | 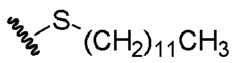 |
| 329 | 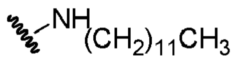 | 330 | 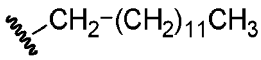 | 331 | 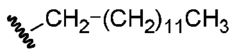 |
| 332 | 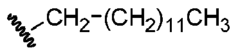 | | | | |
| **$C_{14}$** | | | | | |
| 333 | 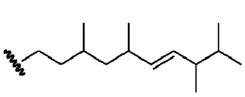 | 334 | 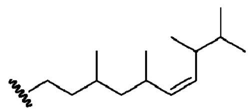 | 335 | 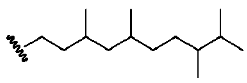 |
| **$C_{15}$** | | | | | |
| 336 | 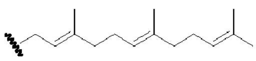 | 337 | 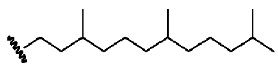 | | |
| **Aryl** | | | | | |
| 338 | 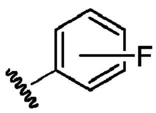 | 339 | 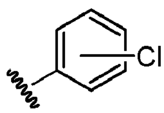 | 340 | 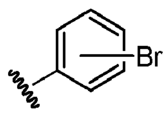 |
| 341 | 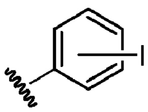 | 342 | 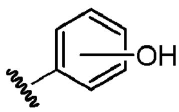 | 343 | 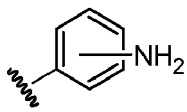 |
| 344 | 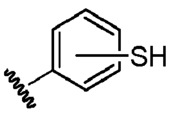 | 345 | 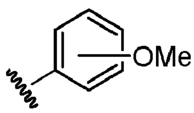 | 346 | 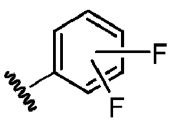 |
| 347 | 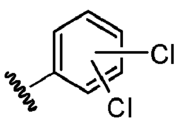 | 348 | 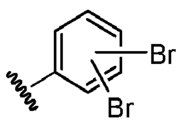 | 349 | 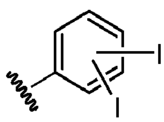 |

(continued)

| number | structure | number | structure | number | structure |
|---|---|---|---|---|---|
| 350 | 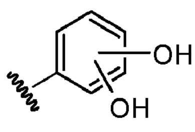 | 351 | 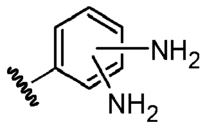 | 352 | 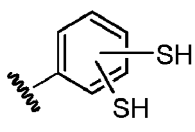 |
| 353 | 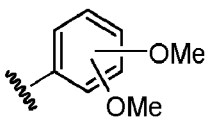 | 354 | 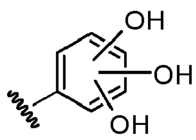 | 355 | 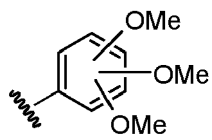 |
| 356 | 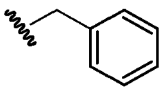 | 357 | 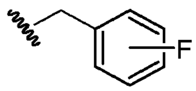 | 358 | 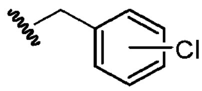 |
| 359 | 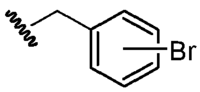 | 360 | 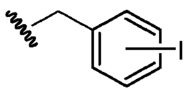 | 361 | 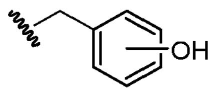 |
| 362 | 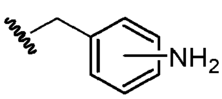 | 363 | 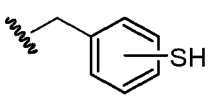 | 364 | 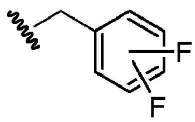 |
| 365 | 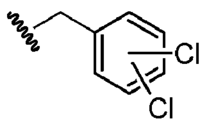 | 366 | 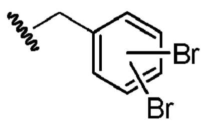 | 367 | 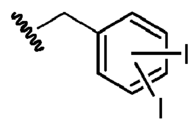 |
| 368 | 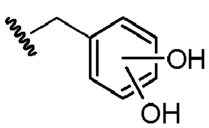 | 369 | 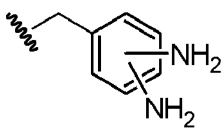 | 370 | 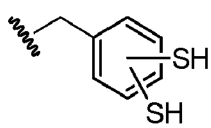 |
| 371 | 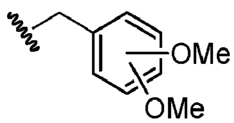 | 372 | 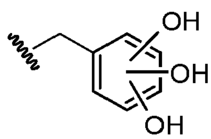 | 373 | 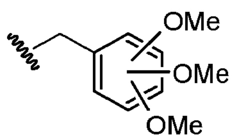 |
| 374 | 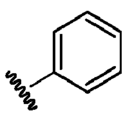 | | | | |

**6.** Pharmaceutical compound for use in the treatment of onychomycoses according to one of the preceding claims, having a structure selected from

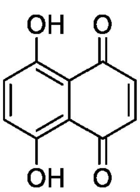 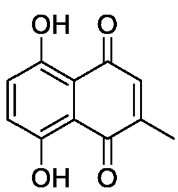 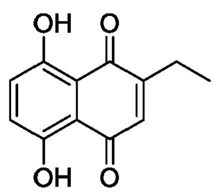

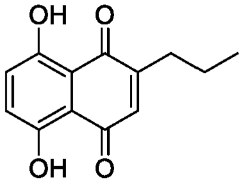

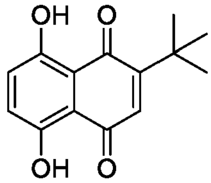

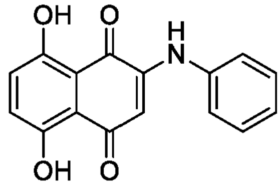

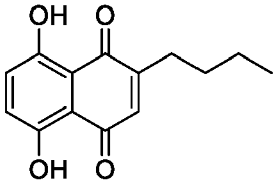

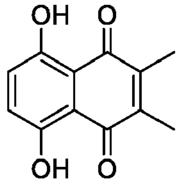

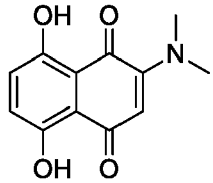

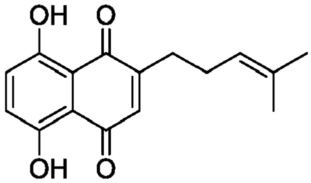

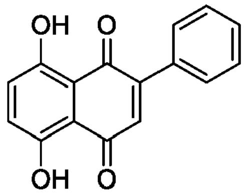

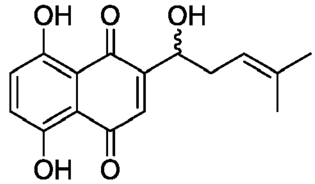

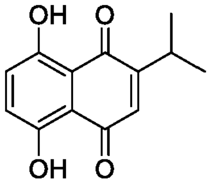

**7.** Pharmaceutical compound for use in the treatment of onychomycoses according to one of the preceding claims, wherein the treatment comprises irradiation of the compound at a wavelength of 500 to 600 nm subsequent to topical application of the compound to a nail.

**8.** Pharmaceutical compound for use in the treatment of onychomycoses according to one of the preceding claims, wherein the treatment comprises penetration of the compound into nail or hoof, without penetration of the compound from the nail into underlying nail matrix.

**9.** Pharmaceutical compound for use in the treatment of onychomycoses according to one of the preceding claims, wherein the onychomycoses is caused by Trichophyton rubrum and/or Candida albicans.

**10.** Pharmaceutical compound for use in the treatment of onychomycoses according to one of the preceding claims, wherein the onychomycosis comprises presence of fungal spores.

**11.** Pharmaceutical compound for use in the treatment of onychomycoses according to one of the preceding claims, wherein the treatment comprises application of the compound to a nail or hoof in the absence of light of a wavelength of 500 to 600 nm.

**12.** Pharmaceutical compound for use in the treatment of onychomycoses according to one of the preceding claims, wherein the treatment subsequent to application of the compound to a nail or hoof comprises removing the compound from skin.

**13.** Process for producing a pharmaceutical compound for use in the treatment of onychomycoses according to one of the preceding claims.

**14.** Pharmaceutical compound for use in medical treatment, the compound comprising structure I

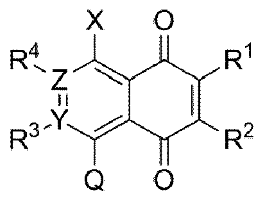

(Structure I),

wherein Z is one of C and N, Y is one of C and N, X is OH or SH or NHR or $NR_2$, Q is OH or SH or NHR or $NR_2$, preferably X is OH and Q is OH, and each of R, R1, R2, R3 and R4 is independently a $C_1$ to $C_{12}$ linear, branched or cyclic alkyl or aryl, or a halogen, and at least one of R3 and R4 is a halogen.

Fig. 1A

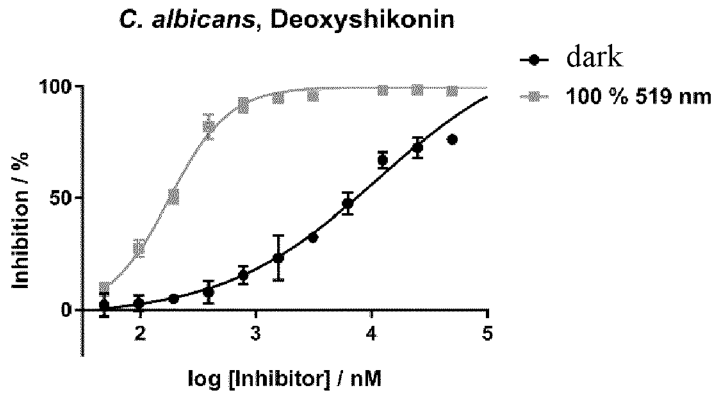


Fig. 1B

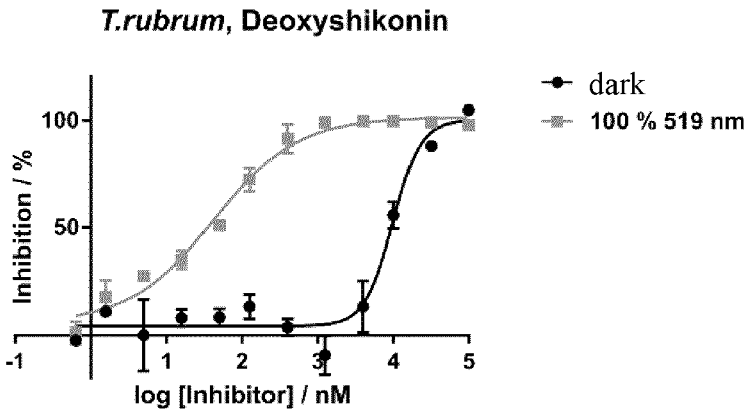


Fig. 2

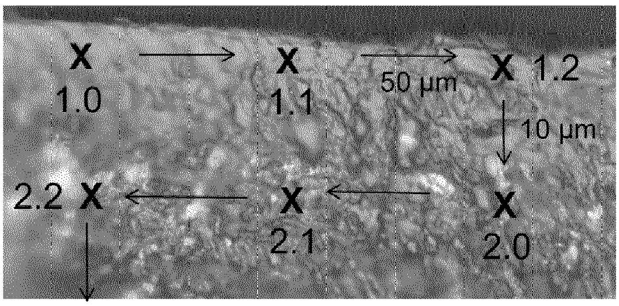

Fig. 3A

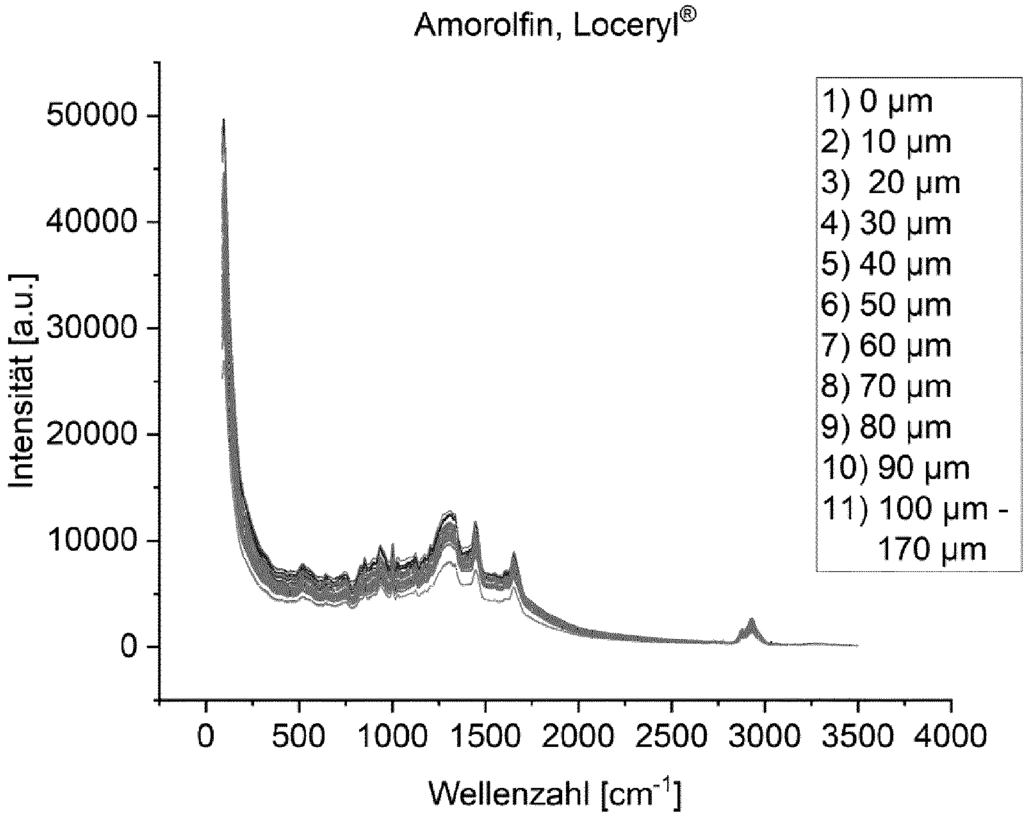
Amorolfin, Loceryl®
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm
Intensität [a.u.]
Wellenzahl [cm-1]
0
10000
20000
30000
40000
50000
500
1000
1500
2000
2500
3000
3500
4000

Fig. 3B

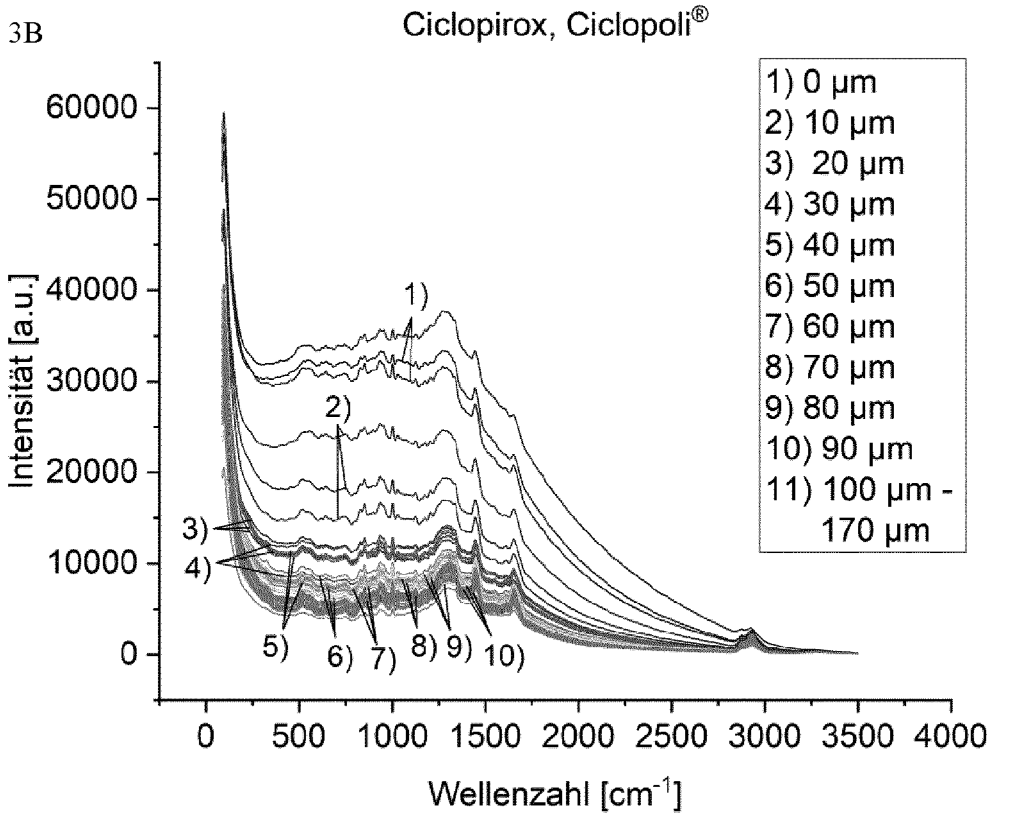
Ciclopirox, Ciclopoli®
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm
1)
2)
3)
4)
5)
6)
7)
8)
9)
10)
Intensität [a.u.]
Wellenzahl [cm-1]
0
10000
20000
30000
40000
50000
60000
500
1000
1500
2000
2500
3000
3500
4000

Fig. 3C

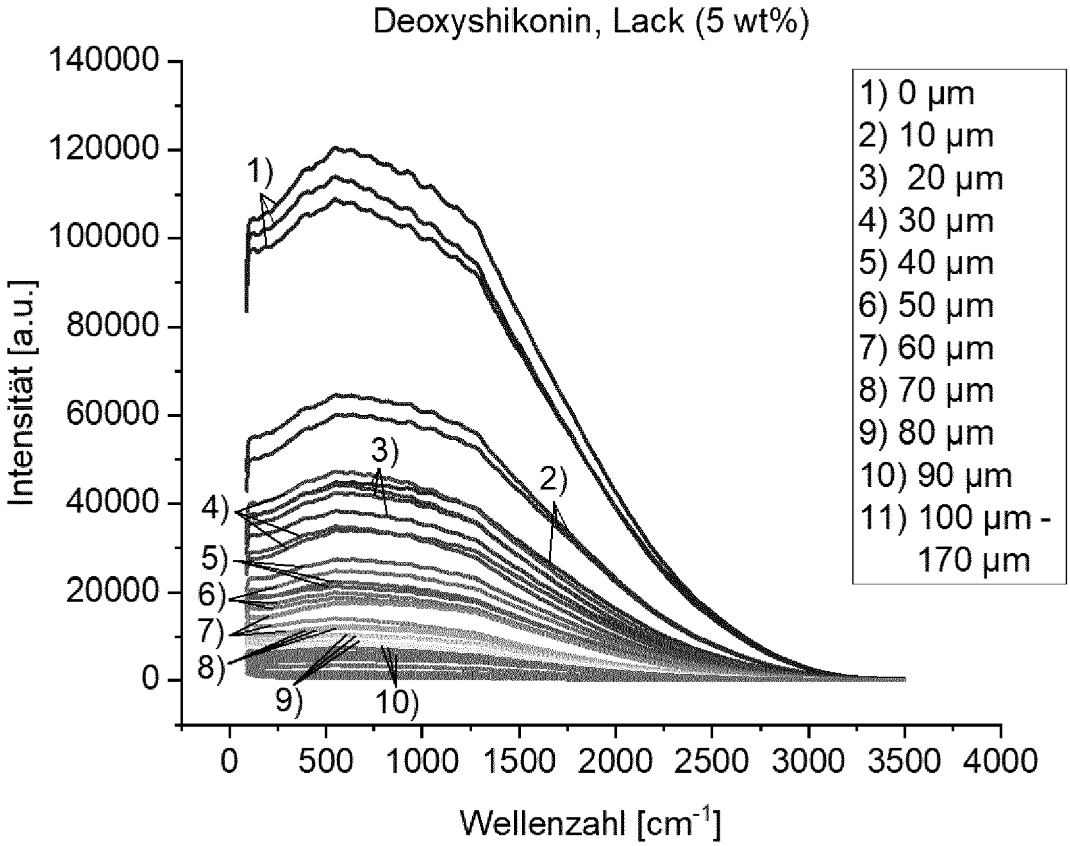
Deoxyshikonin, Lack (5 wt%)
Intensität [a.u.]
Wellenzahl [cm-1]
140000
120000
100000
80000
60000
40000
20000
0
0
500
1000
1500
2000
2500
3000
3500
4000
1)
2)
3)
4)
5)
6)
7)
8)
9)
10)
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm

Europäisches Patentamt
European Patent Office
Office européen des brevets

# EUROPEAN SEARCH REPORT

Application Number
EP 22 18 3652

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DESSOLIN J ET AL: "BROMINATION STUDIES OF THE 2,3-DIMETHYLNAPHTHAZARIN CORE ALLOWING EASY ACCESS TO NAPHTHAZARIN DERIVATIVES", THE JOURNAL OF ORGANIC CHEMISTRY, AMERICAN CHEMICAL SOCIETY, vol. 66, no. 16, 10 August 2001 (2001-08-10), pages 5616-5619, XP001102735, ISSN: 0022-3263, DOI: 10.1021/JO010137N * compounds 10,15 * ----- | 13 | INV. A61K31/122 A61K31/136 A61K31/137 A61P17/00 |
| X | SINGH R ET AL: "synthesis and fungitoxicity of some substituted naphthoquinones and heteroarylquinones", INDIAN JOURNAL OF CHEMISTRY, vol. 28, no. 6, 1 January 1989 (1989-01-01), pages 490-493, XP093005457, DE ISSN: 0376-4699 * page 491, right-hand column, paragraph 1; compound 16 * ----- | 14 | |
| | | | TECHNICAL FIELDS SEARCHED (IPC) |
| A | DE MENEZES HENRIQUE DANTAS ET AL: "Photodynamic treatment with phenothiazinium photosensitizers kills both ungerminated and germinated microconidia of the pathogenic fungiFusarium oxysporum,Fusarium moniliformeandFusarium solani", JOURNAL OF PHOTOCHEMISTRY AND PHOTOBIOLOGY B: BIOLOGY, ELSEVIER SCIENCE S.A., BASEL, CH, vol. 164, 13 September 2016 (2016-09-13), pages 1-12, XP029775291, ISSN: 1011-1344, DOI: 10.1016/J.JPHOTOBIOL.2016.09.008 * the whole document * ----- | 1-14 | A61K A61P |
| | -/-- | | |

The present search report has been drawn up for all claims

| Place of search | Date of completion of the search | Examiner |
|---|---|---|
| Munich | 7 December 2022 | Trifilieff, Sylvie |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 22 18 3652

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUPTA A.K. ET AL: "Onychomycosis: a review", JEADV. JOURNAL OF THE EUROPEAN ACADEMY OF DERMATOLOGY AND VENEREOLOGY., vol. 34, no. 9, 5 June 2020 (2020-06-05), pages 1972-1990, XP55874159, NL ISSN: 0926-9959, DOI: 10.1111/jdv.16394 Retrieved from the Internet: URL:https://onlinelibrary.wiley.com/doi/full-xml/10.1111/jdv.16394> * p. 1981 "novel topical therapies" p. 1982 "lasers" * ----- | 1-14 | |
| A | ZHANG ZIXUAN ET AL: "Shikonin induces necroptosis by reactive oxygen species activation in nasopharyngeal carcinoma cell line CNE-2Z", JOURNAL OF BIOENERGETICS AND BIOMEMBRANES, PLENUM PUBLISHING, NEW YORK, NY, US, vol. 49, no. 3, 25 May 2017 (2017-05-25), pages 265-272, XP036799837, ISSN: 0145-479X, DOI: 10.1007/S10863-017-9714-Z [retrieved on 2017-05-25] * abstract * ----- | 1-14 | |
| | | | TECHNICAL FIELDS SEARCHED (IPC) |

The present search report has been drawn up for all claims

2

| Place of search | Date of completion of the search | Examiner |
|---|---|---|
| Munich | 7 December 2022 | Trifilieff, Sylvie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&
: member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 200219 B1

### Non-patent literature cited in the description

- **PAPAGEORGIOU et al.** *Angew. Chem Int. Ed.,* 1999, 270-300